# EUROPÄISCHE PATENTSCHRIFT

(11) **EP 0 461 483 B1**
(45) Veröffentlichungstag und Bekanntmachung des Hinweises auf die Patenterteilung: **02.11.1995**
(21) Anmeldenummer: 91108882.1
(22) Anmeldetag: 30.05.1991
(51) Int. Cl.: C07C 49/567, C07C 45/68, C07C 49/577, C07C 49/233, C07C 49/813, C07D 303/08, C07D 305/06

(54) **Verfahren zur Herstellung von Benzylketonen**
Method for the production of benzyl ketones
Procédé pour la fabrication de cétones benzyliques

(30) Priorität: 13.06.1990 DE 4018926; 22.09.1990 DE 4030061
(43) Veröffentlichungstag der Anmeldung: 18.12.1991
(73) Patentinhaber: BAYER AG, 51368 Leverkusen (DE)
(72) Erfinder: Himmler, Thomas, Dr., W-5000 Köln 80 (DE); Kraatz, Udo, Dr., W-5090 Leverkusen (DE); Krämer, Wolfgang, Dr., W-5093 Burscheid 2 (DE); Stroech, Klaus, Dr., W-5650 Solingen 19 (DE)

(56) Entgegenhaltungen:
- DE-A- 3 315 510
- DE-A- 3 315 524
- DE-A- 3 315 619
- CHEMISTRY LETTERS, The Chemical Society of Japan, 1981, JP, Seiten 1135 - 1138, T. SATO et al: "Facile synthesis of benzyl ketones by the reductive coupling of benzyl bromide and acyl chlorides in the presence of a palladium catalyst and zinc powder"
- TETRAHEDRON LETTERS, Bd. 24, Nr. 47, 1983, Pergamon Press, Ltd., GB, Seiten 5181 - 5184, E. NEGISHI et al: "Palladium-catalyzed acylation of organozincs and other organometallics as a convenient route to ketones"
- THE JOURNAL OF ORGANIC CHEMISTRY, Bd. 49, Nr. 12, 15. Juni 1984, Columbus, Ohio, US, Seiten 2288 - 2289, R. A. GREY: "A palladium-catalyzed synthesis of ketones from acid chlorides and organozinc compounds"
- THE JOURNAL OF ORGANIC CHEMISTRY, Bd. 53, Nr. 24, 25. November 1988, Columbus, Ohio, US, Seiten 5789 - 5791, S. C. BERK et al: "General approach to highly functionalized benzylic organometallics of zinc and copper"

## Beschreibung

Die vorliegende Erfindung betrifft ein neues Verfahren zur Herstellung von teilweise bekannten Benzylketonen, die als Zwischenprodukte zur Synthese von Wirkstoffen mit fungiziden Eigenschaften verwendet werden können.

Es ist bereits bekannt, daß sich Ketone durch Umsetzung von organischen Säurehalogeniden mit metallorganischen Verbindungen herstellen lassen. Als metallorganische Komponenten kommen dabei Verbindungen in Frage, die sich von Magnesium, Cadmium, Kupfer, Mangan, Quecksilber, Aluminium, Rhodium, Bor oder Silicium ableiten, Nachteilig bei dieser Herstellungsmethode ist jedoch, daß unerwünschte Nebenreaktionen ablaufen, So tritt in vielen Fällen eine Addition der metallorganischen Verbindung an das gebildete Keton ein, Bedingt dadurch wird die Ausbeute an Keton drastisch herabgesetzt. Ungünstig ist auch, daß zum Beispiel metallorganische Verbindungen des Quecksilbers oder Cadmiums stark toxisch sind, und daß z.B. metallorganische Verbindungen des Rhodiums sehr teuer sind. Ein weiterer Nachteil besteht darin, daß die benötigten metallorganischen Verbindungen größtenteils nur über andere metallorganische Stoffe, wie z.B. Grignard-Verbindungen oder lithiumorganische Verbindungen herstellbar sind, Die Variation der einzuführenden Reste ist dadurch stark eingeschränkt.

Weiterhin ist schon bekannt, daß Ketone durch palladiumkatalysierte Kupplung von organischen Säurechloriden mit zinnorganischen Verbindungen zugänglich sind [vergl. J. Org. Chem. 44, (1979) 1613]. Ein Nachteil dieser Methode ist darin zu sehen, daß auch die Darstellung der zinnorganischen Verbindungen über andere metallorganische Zwischenstufen erfolgt. Außerdem gelingt die Synthese der Ketone in kurzen Reaktionszeiten nach dieser Methode nur dann, wenn Hexamethyl-phosphorsäure-triamid als Verdünnungsmittel verwendet wird.

Außerdem ist in der Literatur die Synthese von Ketonen aus organischen Säurechloriden und zinkorganischen Verbindungen in Gegenwart von Palladiumkatalysatoren beschrieben worden [vergl. J. Org. Chem. 49, (1984) 2288 und Tetrahedron Letters 1983, 5181-5184]. Nachteilig an dieser Methode ist, daß auch hier die Herstellung der zinkorganischen Verbindungen mit Hilfe von Grignard-Reagenzien erfolgt. Dadurch ist die Art der einzuführenden Substituenten im wesentlichen auf unsubstituierte Aryl- und Alkyl-Reste beschränkt. Ferner werden für die Durchführung der Reaktion als Verdünnungsmittel technisch nur schwierig zu handhabende Ether oder Gemische von Ethern mit anderen Solventien benötigt.

Im übrigen ist bereits bekannt, daß sich Benzylketone durch palladiumkatalysierte Kupplung von Benzylbromiden mit organischen Säurechloriden in Gegenwart von Zinkpulver herstellen lassen (vergl. Chem. Lett. 1981, 1135 bis 1138). Auch dieses Verfahren ist aber mit einigen Nachteilen verbunden. So müssen relativ große Mengen an Palladiumkatalysatoren verwendet werden. Weiterhin werden die gewünschten Produkte nur in verhältnismäßig niedrigen Ausbeuten erhalten, wenn Chloride von verzweigten aliphatischen Säuren eingesetzt werden.

Schließlich ist ebenfalls schon bekannt, daß Benzyl-phenyl-keton durch palladiumkatalysierte Kupplung von Benzoylchlorid mit Benzylchlorid in Gegenwart von Zinkpulver zugänglich ist (vergl. Chem. Lett. 1981, 1136). Die betreffende Verbindung fällt allerdings nur in sehr niedriger Ausbeute an.

Ferner ist bekannt, daß sich Ketone mit Hilfe von Trimethylsulfonium-Salzen in Gegenwart von Basen in Oxirane überführen lassen (vgl. Ber. 96, 1881-1890 (1963), J. Amer. Chem. Soc. 87, 1353-1364 (1965), DE-OS 3 315 619, DE-OS 3 315 524, DE-OS 3 315 510 und DE-OS 3 315 681). Nachteilig ist jedoch, daß die Ausbeuten in Abhängigkeit von dem jeweils verwendeten Sulfoniumsalz, dem Verdünnungsmittel und der Base stark schwanken.

Darüber hinaus ist auch bekannt, daß bestimmte Oxirane durch Behandlung der entsprechenden Chlorhydrine mit Basen herstellbar sind (vgl. EP-OS 0 297 345). Dieses Verfahren liefert die gewünschten Stoffe zwar in guten Ausbeuten, aber die Synthese der benötigten Ausgangssubstanzen ist relativ aufwendig.

Es wurde nun gefunden, daß man Benzylketone der Formel 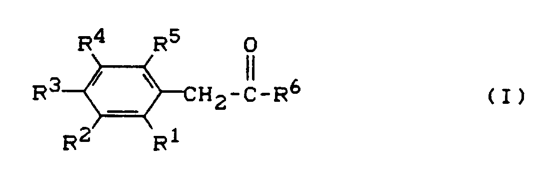
in welcher
- R¹, R², R³, R⁴ und R⁵: unabhängig voneinander für Wasserstoff, Halogen, Alkyl mit 1 bis 6 Kohlenstoffatomen, Halogenalkyl mit 1 bis 6 Kohlenstoffatomen und 1 bis 13 Halogenatomen, Alkoxy mit 1 bis 6 Kohlenstoffatomen, Halogenalkoxy mit 1 bis 6 Kohlenstoffatomen und 1 bis 13 Halogenatomen, Cyano, gegebenenfalls durch Halogen substituiertes Phenoxy oder für Aryl mit 6 bis 10 Kohlenstoffatomen stehen, wobei jeder der Aryl-Reste durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen und/oder Halogenalkoxy mit 1 bis 4 Kohlenstoffatomen und 1 bis 5 Halogenatomen substituiert sein kann, oder
- R¹, R², R³, R⁴ und R⁵: unabhängig voneinander für einen Rest der Formel

-COOR⁷

stehen, worin
- R⁷: für Alkyl mit 1 bis 6 Kohlenstoffatomen, Phenyl oder Benzyl steht,
und
- R⁶: für Alkyl mit 1 bis 18 Kohlenstoffatomen, Halogenalkyl mit 1 bis 18 Kohlenstoffatomen und 1 bis 12 Halogenatomen, Alkenyl mit 2 bis 18 Kohlenstoffatomen, Halogenalkenyl mit 2 bis 18 Kohlenstoffatomen und 1 bis 12 Halogenatomen, Alkoxycarbonylalkyl mit 1 bis 6 Kohlenstoffatomen im Alkylteil und 1 bis 6 Kohlenstoffatomen im Alkoxyteil, für Cycloalkyl mit 3 bis 8 Kohlenstoffatomen oder für Cycloalkenyl mit 5 bis 8 Kohlenstoffatomen steht, wobei jeder der Cycloalkyl- und Cycloalkenyl-Reste substituiert sein kann durch Halogen, Alkyl mit 1 bis 4 Kohlenstoffatomen, Halogenalkyl mit 1 bis 4 Kohlenstoffatomen und 1 bis 7 Halogenatomen, Alkoxy mit 1 bis 4 Kohlenstoffatomen, Halogenalkoxy mit 1 bis 4 Kohlenstoffatomen und 1 bis 7 Halogenatomen Alkenyl mit 2 bis 6 Kohlenstoffatomen und/oder gegebenenfalls durch Halogen und/oder Alkyl mit 1 bis 4 Kohlenstoffatomen substituiertes Phenyl,
oder
- R⁶: für Aryl mit 6 bis 10 Kohlenstoffatomen, für Aralkyl mit 6 bis 10 Kohlenstoffatomen im Arylteil und 1 bis 4 Kohlenstoffatomen im Alkylteil, für Aralkenyl mit 6 bis 10 Kohlenstoffatomen im Arylteil und 2 bis 4 Kohlenstoffatomen im Alkenylteil, oder für Aroxyalkyl mit 6 bis 10 Kohlenstoffatomen im Arylteil und 1 bis 4 Kohlenstoffatomen im Alkylteil steht, wobei jeder dieser Reste im Arylteil substituiert sein kann durch Halogen, Alkyl mit 1 bis 6 Kohlenstoffatomen, Halogenalkyl mit 1 bis 4 Kohlenstoffatomen und 1 bis 5 Halogenatomen, Alkoxy mit 1 bis 4 Kohlenstoffatomer und/oder Halogenalkoxy mit 1 bis 4 Kohlenstoffatomen und 1 bis 5 Halogenatomen,
oder
- R⁶: für einen gegebenenfalls durch Halogen und/oder Alkyl mit 1 bis 4 Kohlenstoffatomen substituierten, heteroaromatischem Rest mit 5 oder 6 Ringgliedern und 1 oder 2 Heteroatomen steht,
oder
- R⁶: für einen Rest der Formel 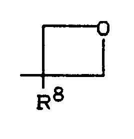 steht, worin
- R⁸: für Alkyl mit 1 bis 4 Kohlenstoffatomen oder für Halogen steht,
durch Umsetzung von Benzylchoriden der Formel 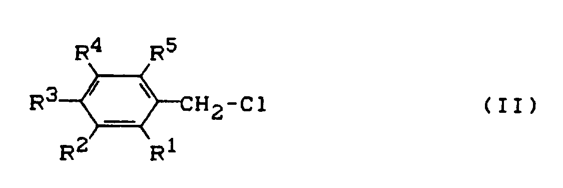
in welcher
- R¹, R², R³, R⁴ und R⁵: die oben angegebenen Bedeutungen haben,
mit Säurechloriden der Formel

R⁶-CO-Cl (III)

in welcher
- R⁶: die oben angegebene Bedeutung hat,
in Gegenwart von Zinkpulver und eines Palladiumkatalysators sowie in Gegenwart eines Verdünnungsmittels erhält, wenn man
a) in einer ersten Stufe ein Benzylchlorid der Formel (II) mit einem Überschuß an Zinkpulver in Gegenwart eines Verdünnungsmittels bei Temperaturen zwischen 30°C und 200°C unter Schutzgasatmosphäre umsetzt, das überschüssige Zinkpulver abtrennt und dann
b) in einer zweiten Stufe das entstandene Benzylderivat der Formel 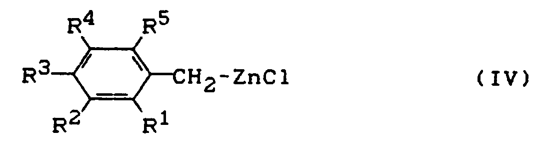 in welcher

- R¹, R², R³, R⁴ und R⁵: die oben angegebenen Bedeutungen haben,
mit einem Säurechlorid der Formel (III) in Gegenwart eines Palladiumkatalysators und in Gegenwart eines Verdünnungsmittels bei Temperaturen zwischen 0°C und 150°C unter Schutzgasatmosphäre umsetzt.

Es ist als äußerst überraschend anzusehen, daß sich Benzylketone der Formel (I) nach dem erfindungsgemäßen, zweistufigen Verfahren in wesentlich höherer Ausbeute herstellen lassen als nach dem entsprechenden vorbekannten Verfahren, bei dem die Umsetzung in einer einzigen Stufe durchgeführt wird, Das Ergebnis ist vor allen Dingen auch deshalb unerwartet, weil aufgrund des bekannten Standes der Technik davon auszugehen war, daß Benzylbromide unter den Verfahrensbedingungen leichter reagieren als Benzylchloride.

Das erfindungsgemäße Verfahren zeichnet sich durch eine Reihe von Vorteilen aus. So ist der Einsatz einer nur sehr geringen Menge an Palladiumkatalysator erforderlich, Weiterhin werden die gewünschten Ketone in hohen Ausbeuten erhalten, Günstig ist außerdem, daß das Verfahren bezüglich der Substituenten im Benzylteil und bezüglich des Säure-Restes breit anwendbar ist.

Verwendet man 2-Chlorbenzylchlorid als Ausgangsstoff und 1-Chlor-cyclopropan-carbonsäurechlorid als Reaktionskomponente, so kann der Verlauf des erfindungsgemäßen Verfahrens durch das folgende Formelschema veranschaulicht werden:

### Erste Stufe

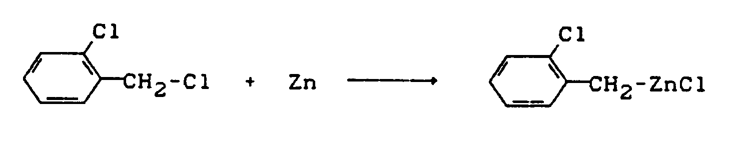

### Zweite Stufe

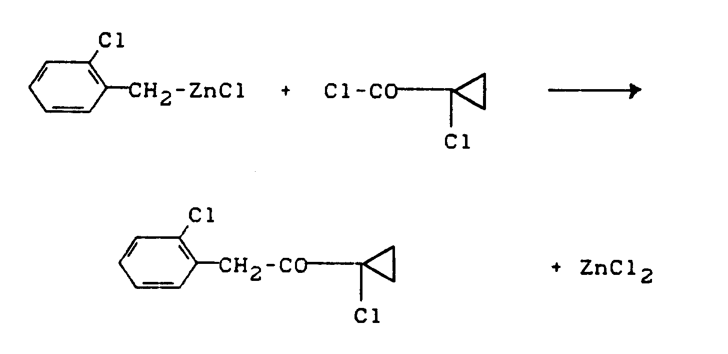

Die bei der Durchführung des erfindungsgemäßen Verfahrens zur Herstellung von Benzylketonen als Ausgangsstoffe benötigten Benzylchloride sind durch die Formel (II) allgemein definiert. In dieser Formel haben die Substituenten R¹, R², R³, R⁴ und R⁵ die oben angegebenen Bedeutungen. Bevorzugt verwendbar sind Benzylchloride der Formel (II), in denen R¹, R², R³, R⁴ und R⁵ unabhängig voneinander für Wasserstoff, Fluor, Chlor, Brom, Alkyl mit 1 bis 4 Kohlenstoffatomen, Halogenalkyl mit 1 bis 4 Kohlenstoffatomen und 1 bis 5 Fluor- und/oder Chloratomen, Alkoxy mit 1 bis 4 Kohlenstoffatomen, Halogenalkoxy mit 1 bis 4 Kohlenstoffatomen und 1 bis 5 Fluor- und/oder Chloratomen, Cyano, gegebenenfalls einfach bis dreifach durch Fluor und/oder Chlor substituiertes Phenoxy, für Phenyl oder Naphthyl stehen, wobei jeder der beiden zuletzt genannten Reste einfach bis dreifach, gleichartig oder verschieden substituiert sein kann durch Fluor, Chlor, Alkyl mit 1 bis 3 Kohlenstoffatomen, Halogenalkyl mit 1 oder 2 Kohlenstoffatomen und 1 bis 5 Fluor- und/oder Chloratomen, Alkoxy mit 1 bis 3 Kohlenstoffatomen oder durch Halogenalkoxy mit 1 oder 2 Kohlenstoffatomen und 1 bis 5 Fluor- und/oder Chloratomen, oder
- R¹, R², R³, R⁴ und R⁵: unabhängig voneinander für einen Rest der Formel

-COOR⁷

stehen, worin
- R⁷: für Alkyl mit 1 bis 4 Kohlenstoffatomen, Phenyl oder Benzyl steht.

Besonders bevorzugt verwendbar sind Benzylchloride der Formel (II), in denen
- R¹: für Wasserstoff, Fluor, Chlor, Brom, Methyl, Ethyl, Trifluormethyl, Trichlormethyl, Methoxy, Ethoxy, Trifluormethoxy, Trichlormethoxy, Cyano, gegebenenfalls durch Fluor und/oder Chlor einfach oder zweifach substituiertes Phenoxy, gegebenenfalls einfach oder zweifach, gleichartig oder verschieden durch Fluor, Chlor, Methyl, Ethyl, Trifluormethyl, Trichlormethyl, Methoxy, Ethoxy, Trifluormethoxy oder Trichlormethoxy substituiertes Phenyl oder für den Rest der Formel

-COOR⁷

steht, worin
- R⁷: für Methyl, Ethyl, Phenyl oder Benzyl steht,
- R²: für Wasserstoff, Fluor, Chlor, Brom, Methyl, Ethyl, Trifluormethyl, Trichlormethyl, Methoxy, Ethoxy, Trifluormethoxy, Trichlormethoxy, Cyano, gegebenenfalls durch Fluor und/oder Chlor einfach oder zweifach substituiertes Phenoxy, gegebenenfalls einfach oder zweifach, gleichartig oder verschieden durch Fluor, Chlor, Methyl, Ethyl, Trifluormethyl, Trichlormethyl, Methoxy, Ethoxy, Trifluormethoxy oder Trichlormethoxy substituiertes Phenyl oder für den Rest der Formel

-COOR⁷

steht, worin
- R⁷: für Methyl, Ethyl, Phenyl oder Benzyl steht,
- R³: für Wasserstoff, Fluor, Chlor, Brom, Methyl, Ethyl, Trifluormethyl, Trichlormethyl, Methoxy, Ethoxy, Trifluormethoxy, Trichlormethoxy, Cyano, gegebenenfalls durch Fluor und/oder Chlor einfach oder zweifach substituiertes Phenoxy, gegebenenfalls einfach oder zweifach, gleichartig oder verschieden durch Fluor, Chlor, Methyl, Ethyl, Trifluormethyl, Trichlormethyl, Methoxy, Ethoxy, Trifluormethoxy oder Trichlormethoxy substituiertes Phenyl oder für den Rest der Formel

-COOR⁷

steht, worin
- R⁷: für Methyl, Ethyl, Phenyl oder Benzyl steht,
- R⁴: für Wasserstoff, Fluor, Chlor, Brom, Methyl, Ethyl, Trifluormethyl, Trichlormethyl, Methoxy, Ethoxy, Trifluormethoxy, Trichlormethoxy, Cyano, gegebenenfalls durch Fluor und/oder Chlor einfach oder zweifach substituiertes Phenoxy, gegebenenfalls einfach oder zweifach, gleichartig oder verschieden durch Fluor, Chlor, Methyl, Ethyl, Trifluormethyl, Trichlormethyl, Methoxy, Ethoxy, Trifluormethoxy oder Trichlormethoxy substituiertes Phenyl oder für den Rest der Formel

-COOR⁷

steht, worin
- R⁷: für Methyl, Ethyl, Phenyl oder Benzyl steht,
und
- R⁵: für Wasserstoff, Fluor, Chlor, Brom, Methyl, Ethyl, Trifluormethyl, Trichlormethyl, Methoxy, Ethoxy, Trifluormethoxy, Trichlormethoxy, Cyano, gegebenenfalls durch Fluor und/oder Chlor einfach oder zweifach substituiertes Phenoxy, gegebenenfalls einfach oder zweifach, gleichartig oder verschieden durch Fluor, Chlor, Methyl, Ethyl, Trifluormethyl, Trichlormethyl, Methoxy, Ethoxy, Trifluormethoxy oder Trichlormethoxy substituiertes Phenyl oder für den Rest der Formel

-COOR⁷

steht, worin
- R⁷: für Methyl, Ethyl, Phenyl oder Benzyl steht.

Die Benzylchloride der Formel (II) sind bekannt oder lassen sich nach prinzipiell bekannten Verfahren in einfacher Weise herstellen.

Die bei der Durchführung des erfindungsgemäßen Verfahrens zur Herstellung von Benzylketonen als Reaktionskomponenten benötigten Säurechloride sind durch die Formel (III) allgemein definiert. In dieser Formel hat der Rest R⁶ die oben angegebenen Bedeutungen. Bevorzugt verwendbar sind Säurechloride der Formel (III), in denen
- R⁶: für Alkyl mit 1 bis 6 Kohlenstoffatomen, Halogenalkyl mit 1 bis 6 Kohlenstoffatomen und 1 bis 6 Fluor-, Chlor- und/oder Bromatomen, Alkenyl mit 2 bis 8 Kohlenstoffatomen, Halogenalkenyl mit 2 bis 8 Kohlenstoffatomen und 1 bis 6 Fluor-, Chlor- und/oder Bromatomen, Alkoxycarbonylalkyl mit 1 bis 4 Kohlenstoffatomen im Alkylteil und 1 bis 4 Kohlenstoffatomen im Alkoxyteil, für Cycloalkyl mit 3 bis 7 Kohlenstoffatomen oder für Cycloalkenyl mit 5 bis 7 Kohlenstoffatomen steht, wobei die Cycloalkyl- und Cycloalkenyl-Reste einfach bis achtfach, gleichartig oder verschieden substituiert sein können durch Fluor, Chlor, Brom, Alkyl mit 1 bis 3 Kohlenstoffatomen, Halogenalkyl mit 1 bis 3 Kohlenstoffatomen und 1 bis 5 Fluor- und/oder Chloratomen, Alkoxy mit 1 bis 3 Kohlenstoffatomen, Halogenalkoxy mit 1 bis 3 Kohlenstoffatomen und 1 bis 5 Fluor- und/oder Chloratomen, durch Alkenyl mit 2 bis 4 Kohlenstoffatomen und/oder gegebenenfalls einfach oder zweifach, gleichartig oder verschieden durch Fluor, Chlor, Methyl und/oder Ethyl substituiertes Phenyl,
oder
- R⁶: für Phenyl, Naphthyl, Phenylalkyl mit 1 bis 4 Kohlenstoffatomen im Alkylteil, Phenylalkenyl mit 2 oder 3 Kohlenstoffatomen im Alkenylteil, oder Phenoxyalkyl mit 1 bis 4 Kohlenstoffatomen im Alkylteil steht, wobei jeder dieser zuvor genannten Reste im Arylteil einfach bis dreifach, gleichartig oder verschieden substituiert sein kann durch Fluor, Chlor, Brom, Alkyl mit 1 bis 3 Kohlenstoffatomen, Halogenalkyl mit 1 bis 3 Kohlenstoffatomen und 1 bis 5 Fluor- und/oder Chloratomen, Alkoxy mit 1 bis 3 Kohlenstoffatomen und/oder Halogenalkoxy mit 1 bis 3 Kohlenstoffatomen und 1 bis 5 Fluor- und/oder Chloratomen,
oder
- R⁶: für einen gegebenenfalls einfach oder zweifach, gleichartig oder verschieden durch Fluor, Chlor, Brom, Methyl und/oder Ethyl substituierten, heteroaromatischen Rest mit 5 oder 6 Ringgliedern und 1 oder 2 Stickstoff-, Sauerstoff- und/oder Schwefelatomen steht,
oder
- R⁶: für einen Rest der Formel 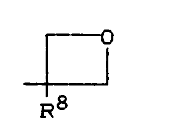 steht, worin
- R⁸: für Methyl, Ethyl, Fluor, Chlor oder Brom steht.

Besonders bevorzugt verwendbar sind Säurechloride der Formel (III), in denen
- R⁶: für Alkyl mit 1 bis 4 Kohlenstoffatomen, Halogenalkyl mit 1 bis 4 Kohlenstoffatomen und 1 bis 5 Fluor-, Chlor- und/oder Bromatomen, Alkenyl mit 2 bis 6 Kohlenstoffatomen, Halogenalkenyl mit 2 bis 6 Kohlenstoffatomen und 1 bis 5 Fluor-, Chlor- und/oder Bromatomen, Alkoxycarbonylalkyl mit 1 bis 3 Kohlenstoffatomen im Alkylteil und 1 bis 3 Kohlenstoffatomen im Alkoxyteil, für Cycloalkyl mit 3 bis 7 Kohlenstoffatomen oder Cycloalkenyl mit 5 bis 7 Kohlenstoffatomen steht, wobei die Cycloalkyl- und Cycloalkenyl-Reste einfach bis achtfach, gleichartig oder verschieden substituiert sein können durch Fluor, Chlor, Brom, Methyl, Ethyl, Propyl, Trifluormethyl, Trichlormethyl, Methoxy, Ethoxy, Trifluormethoxy, Trichlormethoxy, Alkenyl mit 2 oder 3 Kohlenstoffatomen und/oder gegebenenfalls einfach oder zweifach, gleichartig oder verschieden durch Fluor, Chlor, Methyl und/oder Ethyl substituiertes Phenyl steht,
oder
- R⁶: für Phenyl, Naphthyl, Phenylalkyl mit 1 oder 2 Kohlenstoffatomen im Alkylteil, Phenylalkenyl mit 2 Kohlenstoffatomen im Alkenylteil, oder Phenoxyalkyl mit 1 bis 3 Kohlenstoffatomen im Alkylteil steht, wobei jeder der vier zuvor genannten Reste im Arylteil einfach bis dreifach, gleichartig oder verschieden substituiert sein kann durch Fluor, Chlor, Brom, Methyl, Ethyl, Trifluormethyl, Trichlormethyl, Methoxy, Ethoxy, Trifluormethoxy und/oder Trichlormethoxy,
oder
- R⁶: für gegebenenfalls einfach oder zweifach, gleichartig oder verschieden durch Fluor, Chlor, Brom, Methyl und/oder Ethyl substituiertes Pyrrolyl, Pyridinyl, Pyrimidinyl, Furyl oder Thiophenyl steht.
- R⁶: für einen Rest der Formel 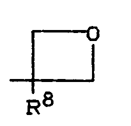 steht, worin
- R⁸: für Methyl, Ethyl, Fluor, Chlor oder Brom steht.

Die Säurechloride der Formel (III) sind bekannt oder lassen sich nach prinzipiell bekannten Verfahren in einfacher Weise herstellen.

Das Zinkpulver kann bei der Durchführung des erfindungsgemäßen Verfahrens zur Herstellung von Benzylketonen in verschiedenen Korngrößen eingesetzt werden. Vorzugsweise verwendbar ist Zinkstaub oder Zinkpulver kleiner Teilchengröße.

Als Palladiumkatalysatoren kommen bei der Durchführung des erfindungsgemäßen Verfahrens zur Herstellung von Benzylketonen alle üblichen Palladium(II)-salze, Palladium(II)-komplexe und Palladium(O)-komplexe in Betracht. Vorzugsweise verwendbar sind Palladium(II)-chlorid, Palladium(II)-acetat, Bis(triphenylphosphin)-palladium(II)-chlorid, Bis(benzonitril)-palladium(II)-chlorid und Tetrakis(triphenylphosphin)-palladium(O).

Als Verdünnungsmittel können bei der Durchführung des erfindungsgemäßen Verfahrens zur Herstellung von Benzylketonen sowohl in der ersten als auch in der zweiten Stufe alle üblichen inerten, organischen Solventien eingesetzt werden. Vorzugsweise verwendbar sind Ether, wie Diethylether, tert.-Butyl-methyl-ether, tert.-Amyl-methyl-ether, Tetrahydrofuran, 2,5-Dimethyl-tetrahydrofuran, Ethylenglykol-dimethylether, Ethylenglykol-tert.-butyl-methyl-ether, Diethylenglykol-diethylether und Dioxan, ferner Nitrile, wie Acetonitril, und außerdem Amide, wie Dimethylformamid und Dimethylacetamid. Die Lösungsmittel werden vorzugsweise in trockener Form eingesetzt.

Als Schutzgase kommen bei der Durchführung des erfindungsgemäßen Verfahrens zur Herstellung von Benzylketonen sowohl in der ersten als auch in der zweiten Stufe alle üblichen inerten Gase in Frage. Vorzugsweise verwendbar sind Helium, Argon und Stickstoff.

Die Reaktionstemperaturen können bei der Durchführung des erfindungsgemäßen Verfahrens zur Herstellung von Benzylketonen sowohl in der ersten als auch in der zweiten Stufe innerhalb eines größeren Bereiches variiert werden. In der ersten Stufe arbeitet man im allgemeinen bei Temperaturen zwischen 30°C und 200°C, vorzugsweise zwischen 50°C und 150°C. in der zweiten Stufe arbeitet man im allgemeinen bei Temperaturen zwischen 0°C und 150°C, vorzugsweise zwischen 20°C und 100°C.

Auch die Reaktionszeiten können im Falle des erfindungsgemäßen Verfahrens zur Herstellung von Benzylketonen sowohl bei der Durchführung der ersten als auch der zweiten Stufe innerhalb eines größeren Bereiches variiert werden. In der ersten Stufe liegen die Reaktionszeiten im allgemeinen zwischen 0,5 und 4 Stunden, vorzugsweise zwischen 1 und 3 Stunden. In der zweiten Stufe liegen die Reaktionszeiten im allgemeinen zwischen 0,1 und 5 Stunden, vorzugsweise zwischen 0,5 und 3 Stunden.

Das erfindungsgemäße Verfahren zur Herstellung von Benzylketonen wird im allgemeinen unter Normaldruck durchgeführt. Es ist jedoch auch möglich, unter vermindertem oder erhöhtem Druck zu arbeiten.

Bei der Durchführung der ersten Stufe des erfindungsgemäßen Verfahrens zur Herstellung von Benzylketonen setzt man das Benzylchlorid der Formel (II) mit einem Überschuß an Zink um. Auf 1 Mol an Benzylchlorid der Formel (II) setzt man im allgemeinen zwischen 1,05 und 3 Mol, vorzugsweise zwischen 1,1 und 2,5 Mol, besonders bevorzugt zwischen 1,25 und 2,0 Mol an Zink ein.

Bei der Durchführung der zweiten Stufe des erfindungsgemäßen Verfahrens zur Herstellung von Benzylketonen kann die Menge an Palladiumkatalysator innerhalb eines bestimmten Bereiches variiert werden. Auf 1 Mol an Säurechlorid der Formel (III) setzt man im allgemeinen zwischen 0,0001 und 1 Mol-%, vorzugsweise zwischen 0,0025 und 0,1 Mol-%, besonders bevorzugt zwischen 0,005 und 0,05 Mol-% an Palladiumkatalysator ein.

Das Verhältnis von Benzylchlorid der Formel (II) zu Säurechlorid der Formel (III) kann bei der Durchführung des erfindungsgemäßen Verfahrens zur Herstellung von Benzylketonen innerhalb eines größeren Bereiches variiert werden. Auf 1 Mol an Säurechlorid der Formel (III) setzt man im allgemeinen zwischen 1,01 und 1,25 Mol, vorzugsweise zwischen 1,05 und 1,2 Mol an Benzylchlorid der Formel (II) ein.

Nach Beendigung der Umsetzung in der ersten Stufe des erfindungsgemäßen Verfahrens zur Herstellung von Benzylketonen kann das überschüssige Zink nach üblichen Methoden abgetrennt werden. Die Entfernung des Zinks kann z.B. durch Filtration oder Zentrifugieren erfolgen, oder auch dadurch geschehen, daß man nach dem Absetzen des Zinks die überstehende Lösung abpumpt.

Bei der Durchführung des erfindungsgemäßen Verfahrens zur Herstellung von Benzylketonen geht man im einzelnen so vor, daß man zunächst das Benzylchlorid der Formel (II) mit Zink in einem geeigneten Verdünnungsmittel unter Schutzgasatmosphäre umsetzt, dann das überschüssige Zink abtrennt, zu der verbleibenden Lösung das Säurechlorid der Formel (III) und den Palladiumkatalysator hinzufügt und bis zur Beendigung der Umsetzung reagieren läßt. Die Aufarbeitung erfolgt nach üblichen Methoden. Im allgemeinen geht man so vor, daß man das Reaktionsgemisch nach gegebenenfalls vorheriger Filtration sowie gegebenenfalls nach dem Verdünnen mit einem mit Wasser wenig mischbaren organischen Lösungsmittel mit Wasser und gegebenenfalls mit verdünnter, wäßriger Mineralsäure versetzt, die organische Phase abtrennt, trocknet und einengt. Das verbleibende Produkt kann nach üblichen Methoden, wie z.B. Destillation oder Umkristallisation, von eventuell noch vorhandenen Verunreinigungen befreit werden.

Die nach dem erfindungsgemäßen Verfahren herstellbaren Benzylketone der Formel (I) sind teilweise bekannt (vergl. Chem. Lett. 1981, 1135 - 1138, EP-OS 0 015 756 und US-PS 4 123 542).

Die Benzylketone der Formel (I) sind wertvolle Zwischenprodukte zur Synthese von Wirkstoffen mit fungiziden Eigenschaften. So lassen sich Azolyl-Derivate der Formel 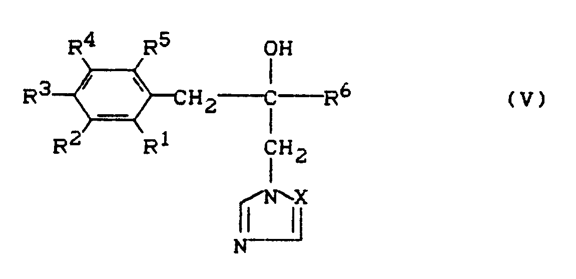
in welcher
- R¹, R², R³, R⁴, R⁵ und R⁶: die oben angegebenen Bedeutungen haben und
- X: für Stickstoff oder eine CH-Gruppe steht,
herstellen, indem man Benzylketone der Formel 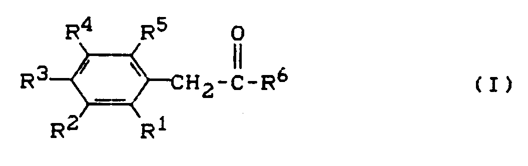
in welcher
- R¹, R², R³, R⁴, R⁵ und R⁶: die oben angegebenen Bedeutungen haben,
entweder
α) mit Dimethyloxosulfonium-methylid der Formel 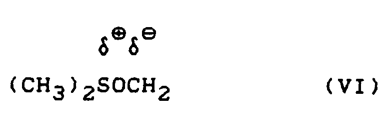 oder
β) mit Dimethylsulfonium-methylid der Formel 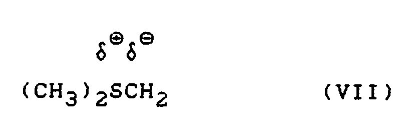
in Gegenwart eines Verdünnungsmittels, wie z.B. tert.-Butanol, Tetrahydrofuran, Dioxan, Toluol oder Dimethylsulfoxid, bei Temperaturen zwischen 10°C und 60°C umsetzt und die dabei entstehenden Oxirane der Formel 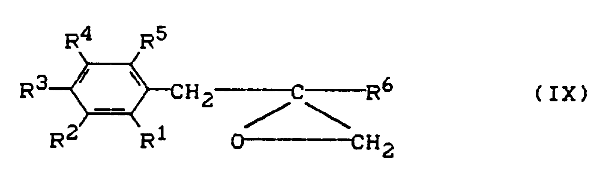
in welcher
- R¹, R², R³, R⁴, R⁵ und R⁶: die oben angegebenen Bedeutungen haben,
mit Azolen der Formel 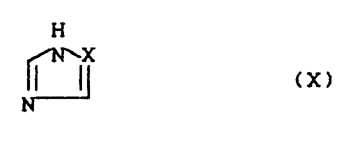
in welcher
- X: die oben angegebene Bedeutung hat,
in Gegenwart eines Saurebindemittels und in Gegenwart eines Verdünnungsmittels bei Temperaturen zwischen 50°C und 150°C umsetzt.

In diesem Zusammenhang wurde gefunden, daß man 2-(2-Chlorbenzyl)-2-(1-chlor-cyclopropyl)-oxiran der Formel 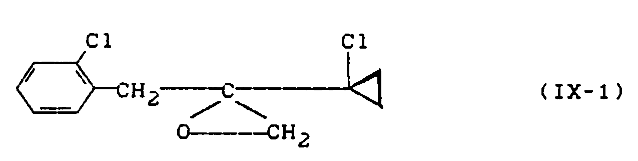
erhält, wenn man 2-Chlorbenzyl-1-chlorcyclopropyl-keton der Formel 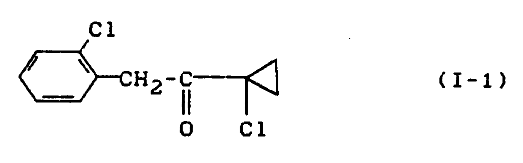
mit Trimethylsulfoxonium-Chlorid der Formel 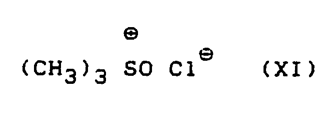
entweder
γ) in Gegenwart eines Alkoholes und in Gegenwart eines Alkalimetallhydroxids oder eines Alkalimetallalkoholates, oder
δ) in Gegenwart von Toluol oder Xylol und in Gegenwart von wäßriger Natronlauge oder Kalilauge
bei Temperaturen zwischen 10°C und 60°C umsetzt.

Es ist als äußerst überraschend zu erachten, daß sich 2-(2-Chlorbenzyl)-2-(1-chlor-cyclopropyl)-oxiran der Formel (IX-1) nach dem obigen Verfahren gerade durch Verwendung des wenig gebräuchlichen Trimethylsulfoxonium-chlorids der Formel (XI) in extrem hoher Ausbeute herstellen läßt, denn beim Einsatz anderer gebräuchlicher Ylid-Bildner liegen die Ausbeuten an dem gewünschten Oxiran drastisch niedriger.

Das Verfahren zur Herstellung von 2-(2-Chlorbenzyl)-2-(1-chlor-cyclopropyl)-oxiran der Formel (IX-1) zeichnet sich durch eine Reihe von Vorteilen aus. So sind die benötigten Reaktionskomponenten in einfacher Weise und auch im technischen Maßstab zugänglich. Ferner bereitet die Durchführung der Synthese und die Isolierung des gewünschten Stoffes keinerlei Probleme. Besonders günstig ist außerdem, daß das Oxiran der Formel (IX-1) nach dieser Methode in extrem hoher Ausbeute und ausgezeichnter Reinheit zugänglich ist.

Der Verlauf des Verfahrens zur Herstellung von 2-(2-Chlorbenzyl)-2-(1-chlor-cyclopropyl)-oxiran der Formel (IX-1) kann durch das folgende Formelschema veranschaulicht werden: 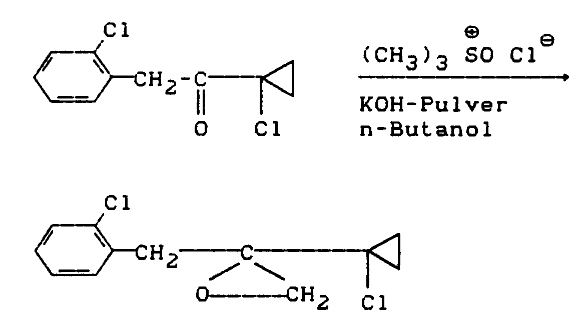
Das bei dem Verfahren zur Herstellung des Oxirans der Formel (IX-1) als Ylid-Bildner benötigte Trimethylsulfoxonium-chlorid der Formel (XI) ist bekannt (vgl. Act. Chem. Scand Ser. B. 28, (1974) 590).

Bei der Durchführung der Variante γ des Verfahrens zur Herstellung des Oxirans der Formel (IX-1) kommt als Verdünnungsmittel vorzugsweise n-Butanol in Betracht. Als Basen kommen vorzugsweise Natrium-hydroxid und Kaliumhydroxid in fester Form in Frage. Sie können als Pellets, Schuppen oder Pulver eingesetzt werden. Außerdem kommen vorzugsweise auch Natriummethylat, Natriumethylat und Natriumbutanolat in Betracht.

Bei der Durchführung der Variante δ des Verfahrens zur Herstellung des Oxirans der Formel (IX-1) arbeitet man im Zweiphasen-System. Als Verdünnungsmittel kommen Toluol und Xylol in Betracht; als Basen können wäßrige Natronlauge oder Kalilauge eingesetzt werden.

Die Reaktionstemperaturen können bei der Durchführung des Verfahrens zur Herstellung des Oxirans der Formel (IX-1) sowohl beim Arbeiten nach der Variante γ als auch der Variante δ innerhalb eines bestimmten Bereiches varriert werden. Im allgemeinen arbeitet man bei Temperaturen zwischen 10°C und 60°C, vorzugsweise zwischen 20°C und 50°C.

Das Verfahren zur Herstellung des Oxirans der Formel (IX-1) wird sowohl beim Arbeiten nach der Variante γ als auch der Variante δ im allgemeinen unter Normaldruck durchgeführt. Es ist jedoch auch möglich, unter erhöhtem oder vermindertem Druck zu arbeiten.

Bei der Durchführung des Verfahrens zur Herstellung des Oxirans der Formel (IX-1) nach der Variante γ setzt man auf 1 Mol an 2-Chlorbenzyl-1-chlorcyclopropyl-keton der Formel (I-1) im allgemeinen 1 bis 3 Mol, vorzugsweise 1 bis 2 Mol an Trimethylsulfoxoniumchlorid der Formel (XI) sowie 1 bis 3 Mol, vorzugsweise 1 bis 2 Mol an Base ein. Beim Arbeiten nach der Variante δ setzt man auf 1 Mol an 2-Chlorbenzyl-1-chlor-cyclopropyl-keton der Formel (I-1) im allgemeinen 1 bis 3 Mol, vorzugsweise 1 bis 2 Mol an Trimethylsulfoxonium-chlorid der Formel (XI) sowie 1 bis 3 Mol, vorzugsweise 1 bis 2 Mol an Base ein. Die Aufarbeitung erfolgt jeweils nach üblichen Methoden. Im allgemeinen geht man so vor, daß man das Reaktionsgemisch nach beendeter Umsetzung mit Wasser versetzt, die organische Phase abtrennt, gegebenenfalls mit Wasser wäscht und einengt und den verbleibenden Rückstand gegebenenfalls destilliert.

Die Durchführung der erfindungsgemäßen Verfahren wird durch die folgenden Beispiele veranschaulicht.

### Beispiel 1

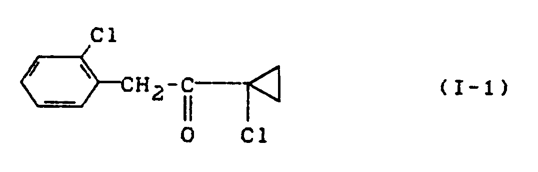

Ein Gemisch aus 32,4 g (0,5 Mol) Zinkpulver, 53,1 g (0,33 Mol) 2-Chlorbenzylchlorid und 375 ml trockenem Ethylenglykol-dimethylether wird unter Stickstoffatmosphäre 2 Stunden unter Rückfluß erhitzt. Anschließend wird das Reaktionsgemisch unter Stickstoff filtriert. Das Filtrat wird mit 41,7 g (0,3 Mol) 1-Chlorcyclopropan-carbonsäurechlorid und 21 mg (0,01 Mol-%) Bis(triphenylphosphin)-palladium(II)-chlorid versetzt und unter Stickstoffatmosphäre 2 Stunden unter Rückfluß erhitzt. Nach dem Abkühlen auf Raumtemperatur wird das Reaktionsgemisch filtriert und unter vermindertem Druck eingeengt. Man nimmt den Rückstand in Toluol auf, schüttelt mit verdünnter, wäßriger Salzsäure aus, trocknet die organische Phase und zieht das Lösungsmittel unter vermindertem Druck ab. Der verbleibende Rückstand wird einer fraktionierten Destillation unterworfen. Dabei erhält man 57,2 g eines Öles, das gemäß Gaschromatogramm zu 95 % aus 2-Chlorbenzyl-1-chlor-cyclopropyl-keton besteht. Die Ausbeute errechnet sich danach zu 79 % der Theorie.

### Beispiel 2

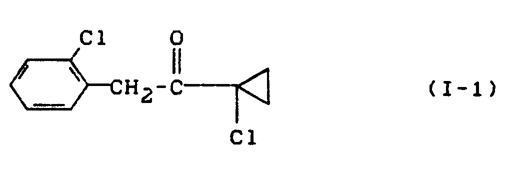

Ein Gemisch aus 2,6 g (40 mMol) Zinkstaub (Teilchengröße 325 mesh), 3,2 g (20 mMol) 2-Chlorbenzylchlorid und 25 ml trockenem Tetrahydrofuran wird unter Stickstoffatmosphäre 1,5 Stunden unter Rückfluß erhitzt. Nach dem Abkühlen auf Raumtemperatur wird das Reaktionsgemisch unter Stickstoff filtriert. Das Filtrat wird mit 2,8 g (20 mMol) 1-Chlorcyclopropan-carbonsäurechlorid und 7,3 mg (0,05 Mol-%) Bis(triphenylphosphin)-palladium(II)-chlorid versetzt und unter Stickstoffatmosphäre 2 Stunden unter Rückfluß erhitzt, Durch gaschromatographische Analyse wird ermittelt, daß 2-Chlorbenzyl-1-chlorcyclopropyl-keton in einer Ausbeute von 85 % der Theorie entstanden ist.

### Beispiel 3

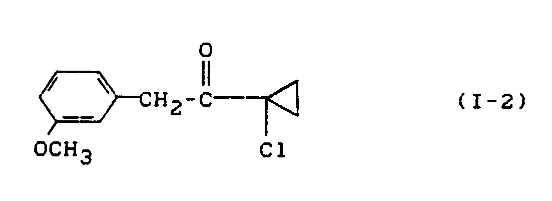

Ein Gemisch aus 3,9 g (60 mMol) Zinkpulver, 4,7 g (30 mMol) 3-Methoxy-benzylchlorid und 45 ml trockenem Ethylenglykol-dimethylether wird unter Stickstoffatmosphäre 3 Stunden unter Rückfluß erhitzt. Anschließend wird das Reaktionsgemisch unter Stickstoff filtriert. Das Filtrat wird mit 3,5 g (25 mMol) 1-Chlorcyclopropan-carbonsäurechlorid und 17,5 mg (0,1 Mol-%) Bis(triphenylphosphin)-palladium(II)-chlorid versetzt und unter Stickstoffatmosphäre 1 Stunde unter Rückfluß erhitzt. Durch gaschromatographische Analyse wird ermittelt, daß 3-Methoxybenzyl-1-chlorcyclopropyl-keton in einer Ausbeute von 59 % der Theorie entstanden ist.

### Beispiel 4

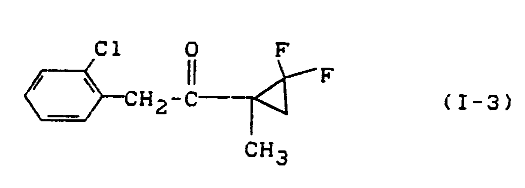

Ein Gemisch aus 6,5 g (100 mMol) Zinkpulver, 10,6 g (66 mMol) 2-Chlorbenzylchlorid und 75 ml trockenem Ethylenglykol-dimethylether wird unter Stickstoffatmosphäre 1 Stunde unter Rückfluß erhitzt. Anschließend wird das Reaktionsgemisch unter Stickstoff filtriert. Das Filtrat wird mit 6,9 g (44,6 mMol) 2,2-Difluor-1-methyl-cyclopropan-carbonsäurechlorid und 21 mg (0,07 Mol-%) Bis(triphenylphosphin)-palladium(II)-chlorid versetzt und unter Stickstoffatmosphäre 1,5 Stunden unter Rückfluß erhitzt. Anschließend wird das Reaktionsgemisch unter Stickstoff filtriert. Man verdünnt das Filtrat mit Toluol, schüttelt nacheinander mit verdünnter, wäßriger Salzsäure und Wasser aus, trocknet die organische Phase und engt durch Abziehen des Lösungsmittels unter vermindertem Druck ein. Der verbleibende Rückstand wird einer Destillation unterworfen. Man erhält auf diese Weise 8,0 g (73 % der Theorie) an 2-Chlorbenzyl-(2,2-difluor-1-methyl)-cyclopropyl-keton in Form eines Öles.
¹H-NMR(200 MHz, CDCl₃) : δ = 1,2-1,4(m;1H), 1,6(m;3H), 2,25-2,45(m;1H), 3,9(d,J ∼ 18Hz;1H), 4,0(d,J ∼ 18Hz;1H), 7,1-7,45(m;4H)ppm.

Nach den zuvor angegebenen Methoden werden auch die in den folgenden Beispielen aufgeführten Benzylketone hergestellt.

### Beispiel 5

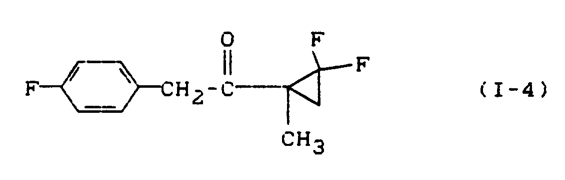

Ausbeute: 85 % der Theorie
¹H-NMR(200 MHz,CDCl₃) : δ = 1,2-1,35(m;1H), 1,5-1,6 (m;3H), 2,25-2,4(m;1H), 3,8(d,J ∼ 17Hz;1H), 3,84 (d, J ∼ 17Hz;1H), 6,9-7,2(m;4H)ppm.

### Beispiel 6

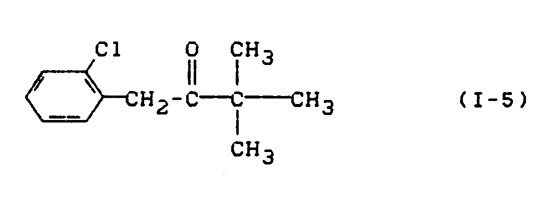

Ausbeute: 74 % der Theorie

### Beispiel 7

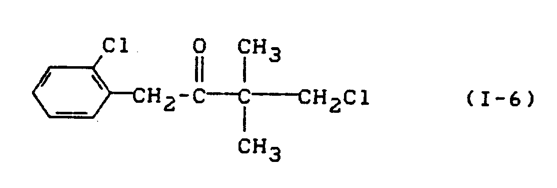

Ausbeute: 60 % der Theorie
¹H-NMR (200MHz, CDCl₃) : δ = 1,34(s;6H), 3,65(s;2H), 3,98(s;2H), 7,2-7,5(m;4H)ppm.

### Beispiel 8

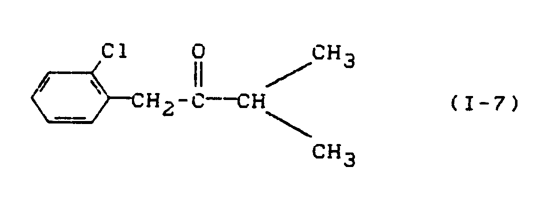

Ausbeute: 91 % der Theorie
¹H-NMR (200MHz, CDCl₃) : δ = 1,13(s;3H), 1,18(s;3H), 2,6-2,8(m;1H), 3,9(s;2H), 7,1-7,4(m;4H)ppm.

### Beispiel 9

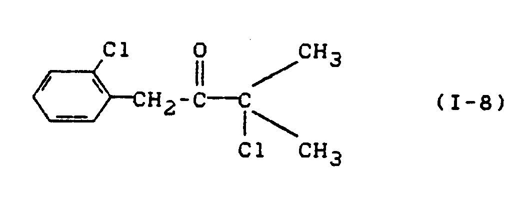

Ausbeute: 85 % der Theorie
¹H-NMR (200MHz, CDCl₃) : δ = 1,78(s;6H), 4,25(s;2H), 7,1-7,4(m;4H)ppm.

### Beispiel 10

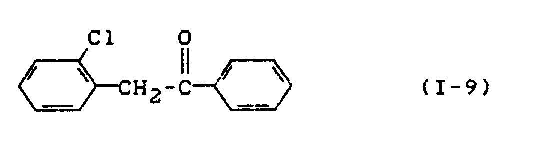

Ausbeute: 66 % der Theorie
Schmelzpunkt: 70°C

### Beispiel 11

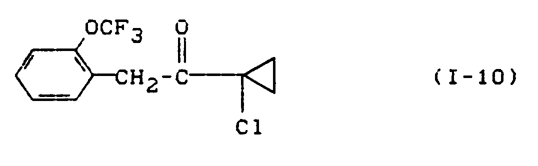

Ausbeute: 82 % der Theorie
¹H-NMR (200MHz, CDCl₃) : δ = 1,3-1,43(m;2H), 1,63-1,73 (m;2H), 4,21(s;2H), 7,1-7,3(m;4H)ppm.

### Beispiel 12

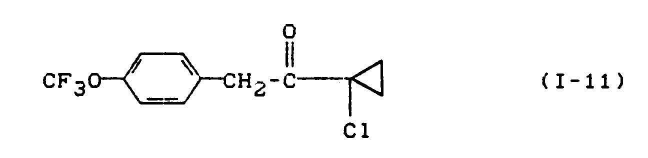

Ausbeute: 67 % der Theorie

### Beispiel 13

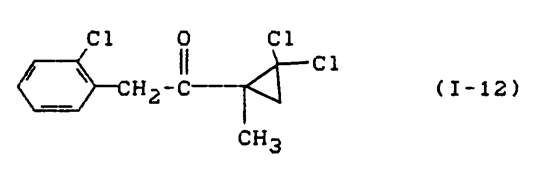

Ausbeute: 91 % der Theorie
¹H-NMR(200 MHz, CDCl₃) : δ = 1,35(d,J ∼ 7Hz;1H), 1,73 (s;3H), 2,29(d,J ∼ 7Hz;1H), 3,9(d,J ∼ 17,5Hz;1H), 4,3 (d,J ∼ 17,5Hz;1H), 7,1-7,4(m;4H)ppm.

### Beispiel 14

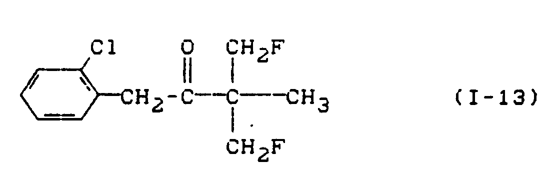

Ausbeute: 93 % der Theorie
¹H-NMR(200 MHz, CDCl₃) : δ = 1,3(t,J ∼ 1Hz;3H), 3,99 (d,J ∼ 20Hz;1H), 4,01(d,J ∼ 20Hz;1H), 4,6 (ddd,J₁∼ 47Hz, J₂∼ 9,5Hz, J₃∼ 2Hz;4H), 7,1-7,4(m;4H)ppm.

### Beispiel 15

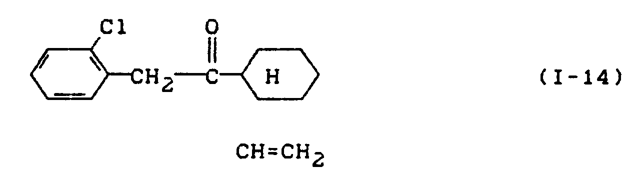

Ausbeute: 70 % der Theorie
¹H-NMR(200 MHz, CDCl₃) : δ = 1,3-2,2(m;10H), 3,90 (d,J ∼ 21Hz;1H), 3,92(d,J ∼ 21Hz;1H), 5,2 (dd,J₁ ∼ 17Hz, J₂ ∼ 1Hz;1H), 5,3(dd,J₁ ∼ 11Hz, J₂ ∼ 1Hz;1H), 5,8(dd,J₁ ∼ 17Hz, J₂ ∼ 11Hz;1H), 7,0-7,4 (m;4H)ppm.

### Beispiel 16

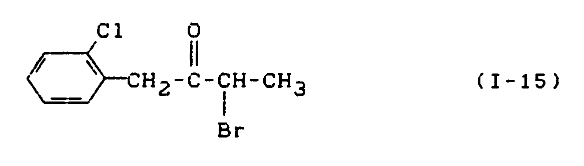

Ausbeute: 86 % der Theorie
¹H-NMR(200 MHz, CDCl₃) : δ = 1,75(d,J ∼ 7Hz;3H), 4,13 (s;2H), 4,5(q,J ∼ 7Hz;1H), 7,2-7,4(m;4H)ppm.

### Beispiel 17

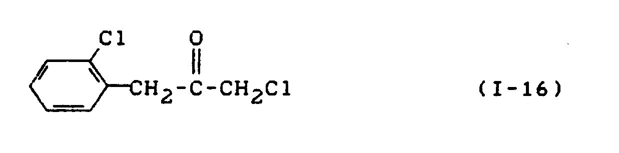

Ausbeute: 90 % der Theorie
¹H-NMR(200 MHz, CDCl₃) : δ = 3,99(s;2H), 4,18(s;2H), 7,2-7,5(m;4H)ppm.

### Beispiel 18

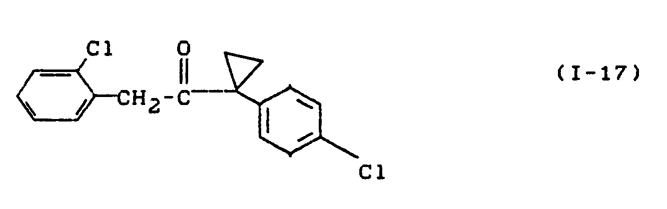

Ausbeute: 95 % der Theorie
¹H-NMR (200 MHz, CDCl₃): δ = 1,15-1,25 (m; 2H), 1,65-1,75 (m, 2H), 3,68 (s; 2H), 7,0-7,4 (m; 8H) ppm.

### Beispiel 19

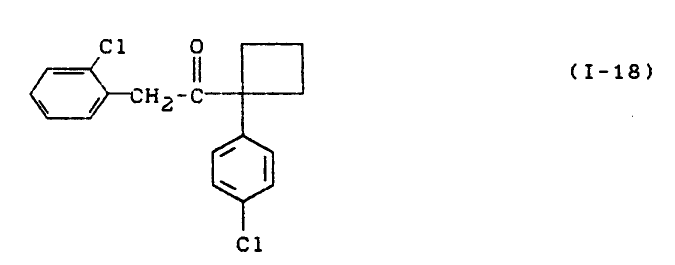

Ausbeute: 80 % der Theorie
¹H-NMR (200 MHz, CDCl₃): δ = 1,8-2,05 (m; 2H), 2,3-2,55 (m; 2H), 2,8-3,0 (m; 2H), 3,63 (s; 2H), 6,9-7,4 (m; 8H) ppm.

### Beispiel 20

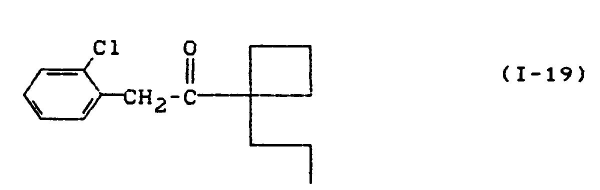

Ausbeute: 88 % der Theorie
¹H-NMR (200 MHz, CDCl₃): δ = 0,8-1,0 (t, 3H), 1,1-1,3 (m; 2H), 1,7-2,0 (m; 6H), 2,35-2,6 (m; 2H), 3,81 (s; 2H), 7,1-7,4 (m; 4H) ppm.

### Beispiel 21

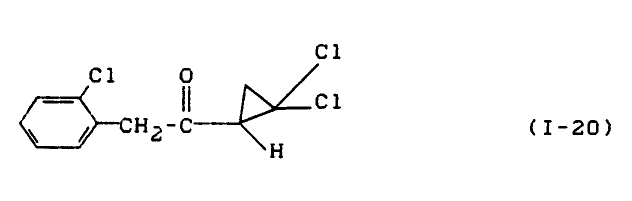

Ausbeute: 89 % der Theorie
¹H-NMR (200 MHz, CDCl₃): δ = 1,8 (dd, J₁ ∼ 9 Hz, J₂ ∼ 6 Hz; 1H), 2,15 (dd, J₁ ∼ 7,5 Hz, J₂ ∼ 6 Hz, 1H), 2,8 (dd, J₁ ∼ 9 Hz, J₁ ∼ 7,5 Hz, 1H), 4,05 (s, 2H), 7,2-7,5 (m, 4H) ppm.

### Beispiel 22

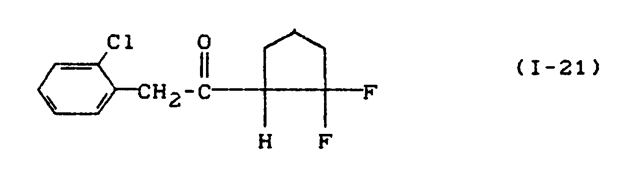

Ausbeute: 76 % der Theorie
¹H-NMR (200 MHz, CDCl₃): δ = 1,6-2,4 (m; 6H), 3,25-3,5 (m; 1H), 3,97 (d, J ∼ 16 Hz; 1H), 4,03 (d, J ∼ 16 Hz; 1H), 7,1-7,5 (m; 4H) ppm.

### Beispiel 23

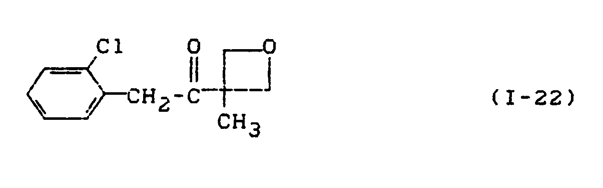

Ausbeute: 73 % der Theorie
¹H-NMR (270 MHz, CDCl₃): δ = 1,45 (s, 3H), 3,85 (s, 4H), 4,06 (s, 2H), 7,0-7,4 (m, 5H) ppm.

### Beispiel 24

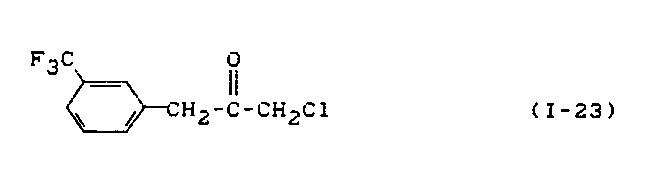

Ausbeute: 80 % der Theorie
¹H-NMR (200 MHz, CDCl₃): δ = 3,95 (s, 2H), 4,12 (s, 2H), 7,35-7,6 (m, 4H) ppm.
Fp.: 42°C

### Beispiel 25

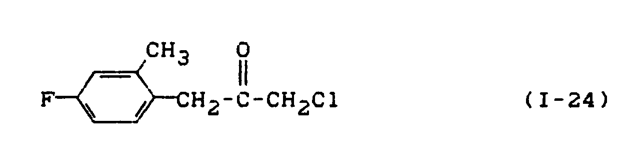

Ausbeute: 55 % der Theorie
¹H-NMR (200 MHz, CDCl₃): δ = 2,20 (s, 3H), 3,84 (s, 2H), 4,08 (s, 2H), 6,8-7,15 (m, 3H) ppm.
Kp.: 99-103°C/1 mbar

### Beispiel 26

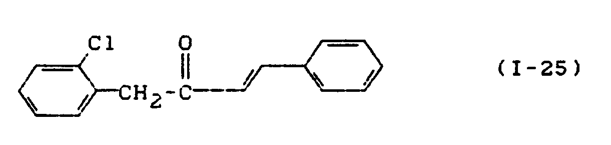

Ausbeute: 40 % der Theorie
¹H-NMR (200 MHz, CDCl₃): δ = 4,08 (s, 2H), 6,78 (d, J = 16 Hz; 1H), 7,2-7,6 (m, 9H), 7,65 (d, J = 16 Hz; 1H) ppm

### Beispiel 27

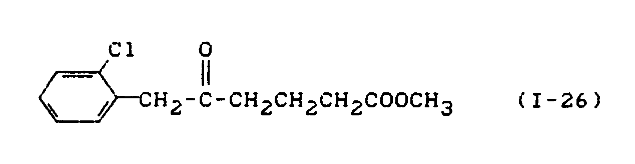

Ausbeute: 80 % der Theorie
¹H-NMR (200 MHz, CDCl₃): δ = 1,8-2,0 (m, 2H), 2,33 (t, J = 7 Hz, 2H), 2,55 (t, J = 7 Hz, 2H), 3,63 (s, 3H), 3,80 (s, 2H), 7,1-7,4 (m, 4H) ppm

### Beispiel 28

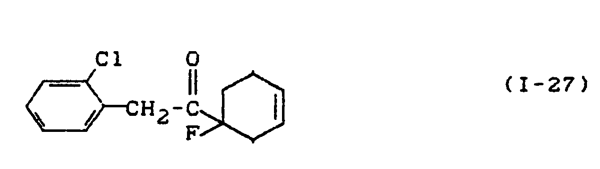

Ausbeute: 80 % der Theorie
¹H-NMR (200 MHz, CDCl₃): δ = 1,65-2,8 (m, 6H), 4,0-4,3 (m, 2H), 5,5-5,9 (m, 2H), 7,1-7,5 (m, 4H) ppm

### Beispiel 29

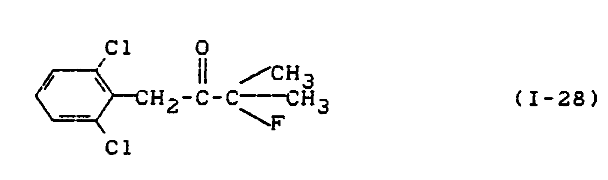

Ausbeute: 59 % der Theorie
¹H-NMR (200 MHz, CDCl₃): δ = 1,56 (d, J = 21 Hz, 6H), 4,35 (m, 2H), 7,1-7,35 (m, 3H) ppm.

### Beispiel 30

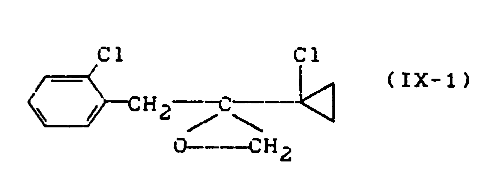

Ein Gemisch aus 134 g (0,585 Mol) 2-Chlorbenzyl-1-chlorcyclopropyl-keton und 35,5 g (0,64 Mol) Kaliumhydroxid-Pulver in 700 ml absolutem n-Butanol wird unter Rühren bei Raumtemperatur portionsweise mit 82,2 g (0,64 Mol) Trimethylsulfoxonium-chlorid versetzt. Nach beendeter Zugabe wird das Reaktionsgemisch noch 3 Stunden bei 35°C gerührt und dann in 500 ml Wasser eingerührt. Man trennt die organische Phase ab, wäscht einmal mit 500 ml Wasser, engt unter vermindertem Druck ein und destilliert den verbleibenden Rückstand im Vakuum. Auf diese Weise erhält man 134,6 g eines Produktes, das zu 92,6 % aus 2-(2-Chlorbenzyl)-2-(1-chlorcyclopropyl)-oxiran besteht und einen Siedepunkt von 88-90°C bei einem Druck von 0,2 mbar aufweist. Die Ausbeute errechnet sich danach zu 87,7 % der Theorie.

### Beispiel 31

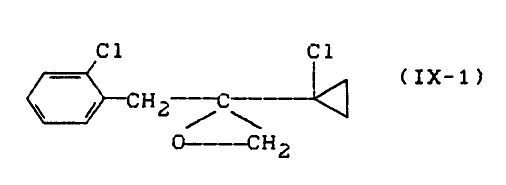

Zu einer Suspension von 171,0 g (0,75 Mol) 2-Chlorbenzyl-1-chlorcyclopropyl-keton und 104,0 g (0,81 Mol) Trimethylsulfoxonium-chlorid in 1000 ml Toluol werden bei 20°C unter Rühren 507 g (5,7 Mol) 45 Gew.-%ige, wäßrige Natronlauge getropft. Dabei steigt die Temperatur des Reaktionsgemisches bis auf 32°C an. Nach beendeter Zugabe wird das Reaktionsgemisch noch 4 Stunden bei 30°C gerührt und dann unter Kühlung mit 600 ml Wasser versetzt, Man trennt die organische Phase ab, und extrahiert die wäßrige Phase noch zweimal mit Toluol, Die vereinigten organischen Phasen werden nach dem Trocknen über Magnesiumsulfat unter verminderten Druck eingeengt. Man erhält auf diese Weise 175,6 g eines öligen Produktes, das zu 90,5 % aus 2-(2-Chlorbenzyl)-2-(1-chlor-cyclopropyl)-oxiran besteht. Die Ausbeute errechnet sich danach zu 87,3 % der Theorie.

### Vergleichsbeispiel 1

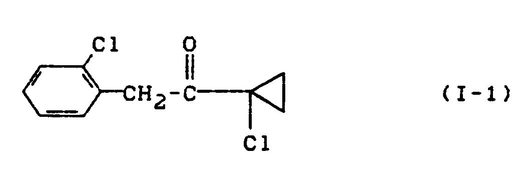

Ein Gemisch aus 2,6 g (40 mMol) Zinkstaub (Teilchengröße 325 mesh), 3,2 g (20 mMol) 2-Chlorbenzylchlorid und 25 ml trockenem Tetrahydrofuran wird unter Stickstoffatmosphäre 1,5 Stunden unter Rückfluß erhitzt. Danach wird das Reaktionsgemisch mit 2,8 g (20 mMol) 1-Chlorcyclopropan-carbonsäurechlorid und 7,3 mg (0,05 Mol-%) Bis-(triphenylphosphin)-palladium(II)-chlorid versetzt und unter Stickstoffatmosphäre 2 Stunden unter Rückfluß erhitzt. Durch gaschromatographische Analyse wird ermittelt, daß 2-Chlorbenzyl-1-chlorcyclopropyl-keton in einer Ausbeute von 50 % der Theorie entstanden ist.

### Vergleichsbeispiel 2

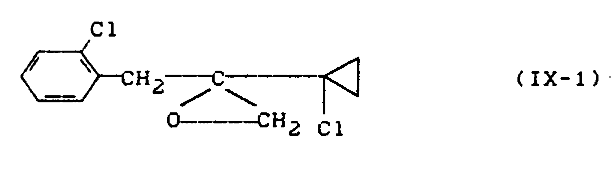

Zu einer Suspension von 17,2 g (0,078 Mol) Trimethylsulfoxonium-iodid in 70 ml Dimethylsulfoxid fügt man bei Raumtemperatur 8,7 g (0,078 Mol) Kalium-tert.-butylat hinzu und rührt 30 Minuten bei Raumtemperatur. Anschließend werden 15 g (0,066 Mol) 2-Chlorbenzyl-1-chlorcyclopropyl-keton zugesetzt. Nach beendeter Zugabe wird das Reaktionsgemisch 4 Stunden bei 40°C gerührt und dann nach dem Abkühlen auf Wasser gegossen. Das entstehende Gemisch wird mehrfach mit Methylenchlorid extrahiert. Die vereinigten organischen Phasen werden mit Wasser gewaschen und nach dem Trocknen über Magnesiumsulfat unter verminderten Druck eingeengt. Man erhält auf diese Weise 16,6 g eines öligen Produktes, das zu 44 % aus dem 2-(2-Chlorbenzyl)-2-(1-chlor-cyclopropyl)-oxiran besteht. Die Ausbeute errechnet sich danach zu 45,5 % der Theorie.

### Vergleichsbeispiel 3

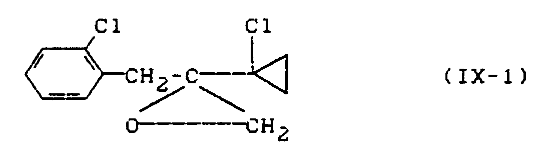

Ein Gemisch aus 10,0 g (0,044 Mol) 2-Chlorbenzyl-1-chlor-cyclopropyl-keton und 2,9 g (0,052 Mol) Kaliumhydroxidpulver in 45 ml absolutem tert.-Butanol wird unter Rühren bei Raumtemperatur portionsweise mit 11,5 g (0,052 Mol) Trimethylsulfoxonium-iodid versetzt. Nach beendeter Zugabe wird das Reaktionsgemisch noch 16 Stunden bei 40°C gerührt und dann in 100 ml Wasser eingerührt. Man extrahiert mit 100 ml Dichlormethan, trennt die organische Phase ab, wäscht einmal mit 60 ml Wasser, engt unter vermindertem Druck ein und destilliert den verbleibenden Rückstand im Vakuum. Auf diese Weise erhält man 9 g eines Produktes, das zu 15 % aus 2-(2-Chlorbenzyl)-2-(1-chlorcyclopropyl)-oxiran besteht. Die Ausbeute errechnet sich danach zu 13 % der Theorie.

### Vergleichsbeispiel 4

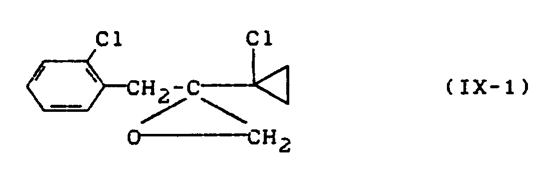

Zu einer Lösung von 13,0 g (0,069 Mol) Trimethylsulfoxonium-methylsulfat in 53 ml Acetonitril werden bei 20°C unter Rühren 4,2 g (0,078 Mol) Natriummethylat gegeben. Nach beendeter Zugabe wird noch 30 Minuten bei 20°C gerührt und dann mit 10 g (0,044 Mol)2-Chlorbenzyl-1-chlorcyclopropyl-keton versetzt. Man rührt 16 Stunden bei 20 bis 30°C und verdünnt dann mit Wasser. Das entstehende Gemisch wird mit Methylenchlorid extrahiert. Die vereinigten organischen Phasen werden mit Wasser gewaschen, über Magnesiumsulfat getrocknet und unter vermindertem Druck eingeengt. Man erhält auf diese Weise 7,5 g eines öligen Produktes, das gemäß gaschromatographischer Analayse zu 38 % aus 2-Chlorbenzyl-1-chlorcyclopropyl-keton und zu 3,6 % aus 2-(2-Chlorbenzyl)-2-(1-chlorcyclopropyl)-oxiran besteht. Die Ausbeute errechnet sich danach zu 2,5 % der Theorie.

### Vergleichsbeispiel 5

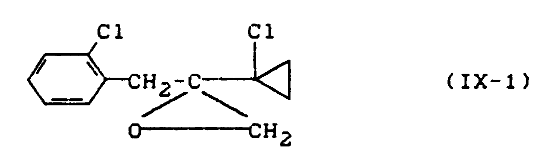

Zu einer Suspension von 17,2 g (0,11 Mol) Trimethylsulfonium-bromid in 70 ml Dimethylsulfoxid werden bei 20°C unter Rühren portionsweise 8,9 g (0,079 Mol) Kalium-tert.-butylat gegeben. Nach beendeter Zugabe wird noch 20 Minuten bei 20°C gerührt und dann mit 15 g (0,0655 Mol) 2-Chlorbenzyl-1-chlorcyclopropyl-keton versetzt. Man rührt 4 Stunden bei 40°C und dann noch 16 Stunden bei 20°C und verdünnt danach mit Wasser. Das entstehende Gemisch wird mehrfach mit Methylenchlorid extrahiert. Die vereinigten organischen Phasen werden mit Wasser gewaschen, über Magnesiumsulfat getrocknet und unter vermindertem Druck eingeengt. Man erhält auf diese Weise 15,5 g eines öligen Produktes, das gemäß gaschromatographischer Analyse zu 77 % aus 2-Chlorbenzyl-1-chlorcyclopropyl-keton und zu 5 % aus 2-(2-Chlorbenzyl)-2-(1-chlorcyclopropyl)-oxiran besteht. Die Ausbeute errechnet sich danach zu 4,9 % der Theorie.

### Vergleichsbeispiel 6

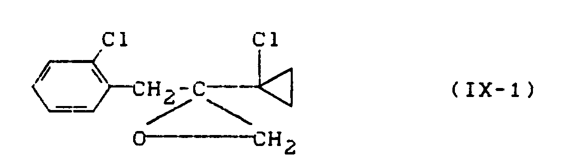

Zu einer Mischung aus 10 g (0,044 Mol) 2-Chlorbenzyl-1-chlorcyclopropyl-keton, 50 mg Ölsäure-polyglykolether und 0,1 ml Methanol in 11 ml Toluol werden bei 20°C unter Rühren 7,5 g (0,13 Mol) Kaliumhydroxid-Pulver gegeben. Anschließend erwärmt man auf 40°C und tropft unter Rühren eine Lösung von 6,6 g (0,059 Mol) Trimethylsulfonium-chlorid in einem Gemisch aus 1,5 ml Wasser und 0,2 ml Diethylenglykol hinzu. Das Reaktionsgemisch wird 16 Stunden bei 40°C gerührt und danach mit Wasser verdünnt. Das entstehende Gemisch wird mit Toluol extrahiert. Die vereinigten organischen Phasen werden unter vermindertem Druck eingeengt. Man erhält auf diese Weise 11,3 g eines öliges Produkte, das zu 12 % aus 2-(2-Chlorbenzyl)-2-(1-Chlor-cyclopropyl)-oxiran besteht, Die Ausbeute errechnet sich danach zu 13 % der Theorie.

### Verwendungsbeispiel

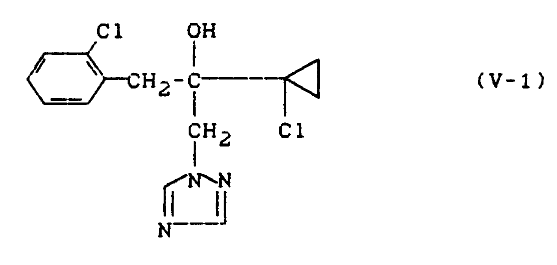

Zu einem Gemisch von 2,2 g 1,2,4-Triazol und 0,23 g Kalium-tert.-butylat in 10 ml trockenem Dimethylformamid wird unter Stickstoffatmosphäre und unter Rühren bei 80°C eine Lösung von 2,84 g 2-(2-Chlorphenyl)-2-(1-chlor-cyclopropyl)-oxiran in 3 ml trockenem Dimethylformamid getropft. Nach beendeter Zugabe wird das Reaktionsgemisch noch 8 Stunden auf 80°C erhitzt und danach unter vermindertem Druck eingeengt. Der verbleibende Rückstand wird in Essigester gelöst. Die dabei entstehende Losung wird mit Wasser ausgeschüttelt, getrocknet und unter vermindertem Druck eingeengt. Der verbleibende Rückstand wird mit Methylenchlorid als Laufmittel über eine Kieselgel-Säule chromatographiert. Nach dem Eindampfen des Eluates erhält man 1,77 g an 3-(2-Chlorphenyl)-2-(1-chlor-cyclopropyl)-1-(1,2,4-triazol-1-yl)-propan-2-ol in Form einer Festsubstanz vom Schmelzpunkt 107°C.

## Patentansprüche

1. Verfahren zur Herstellung von Benzylketonen der Formel 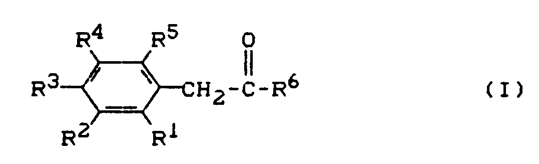 in welcher
R¹, R², R³, R⁴ und R⁵ unabhängig voneinander für Wasserstoff, Halogen, Alkyl mit 1 bis 6 Kohlenstoffatomen, Halogenalkyl mit 1 bis 6 Kohlenstoffatomen und 1 bis 13 Halogenatomen, Alkoxy mit 1 bis 6 Kohlenstoffatomen, Halogenalkoxy mit 1 bis 6 Kohlenstoffatomen und 1 bis 13 Halogenatomen, Cyano, gegebenenfalls durch Halogen substituiertes Phenoxy oder für Aryl mit 6 bis 10 Kohlenstoffatomen stehen, wobei jeder der Aryl-Reste durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen und/oder Halogenalkoxy mit 1 bis 4 Kohlenstoffatomen und 1 bis 5 Halogenatomen substituiert sein kann, oder
R¹, R², R³, R⁴ und R⁵ unabhängig voneinander für einen Rest der Formel
-COOR⁷
stehen, worin
R⁷ für Alkyl mit 1 bis 6 Kohlenstoffatomen, Phenyl oder Benzyl steht,
und
R⁶ für Alkyl mit 1 bis 18 Kohlenstoffatomen, Halogenalkyl mit 1 bis 18 Kohlenstoffatomen und 1 bis 12 Halogenatomen, Alkenyl mit 2 bis 18 Kohlenstoffatomen, Halogenalkenyl mit 2 bis 18 Kohlenstoffatomen und 1 bis 12 Halogenatomen, Alkoxycarbonylalkyl mit 1 bis 6 Kohlenstoffatomen im Alkylteil und 1 bis 6 Kohlenstoffatomen im Alkoxyteil, für Cycloalkyl mit 3 bis 8 Kohlenstoffatomen oder für Cycloalkenyl mit 5 bis 8 Kohlenstoffatomen steht, wobei jeder der Cycloalkyl- und Cycloalkenyl-Reste substituiert sein kann durch Halogen, Alkyl mit 1 bis 4 Kohlenstoffatomen, Halogenalkyl mit 1 bis 4 Kohlenstoffatomen und 1 bis 7 Halogenatomen, Alkoxy mit 1 bis 4 Kohlenstoffatomen, Halogenalkoxy mit 1 bis 4 Kohlenstoffatomen und 1 bis 7 Halogenatomen, Alkenyl mit 2 bis 6 Kohlenstoffatomen und/oder gegebenenfalls durch Halogen und/oder Alkyl mit 1 bis 4 Kohlenstoffatomen substituiertes Phenyl,
oder
R⁶ für Aryl mit 6 bis 10 Kohlenstoffatomen, für Aralkyl mit 6 bis 10 Kohlenstoffatomen im Arylteil und 1 bis 4 Kohlenstoffatomen im Alkylteil steht, für Aralkenyl mit 6 bis 10 Kohlenstoffatomen im Arylteil und 2 bis 4 Kohlenstoffatomen im Alkenylteil steht, oder für Aroxyalkyl mit 6 bis 10 Kohlenstoffatomen im Arylteil und 1 bis 4 Kohlenstoffatomen im Alkylteil steht, wobei jeder dieser Reste im Arylteil substituiert sein kann durch Halogen, Alkyl mit 1 bis 6 Kohlenstoffatomen, Halogenalkyl mit 1 bis 4 Kohlenstoffatomen und 1 bis 5 Halogenatomen, Alkoxy mit 1 bis 4 Kohlenstoffatomen und/oder Halogenalkoxy mit 1 bis 4 Kohlenstoffatomen und 1 bis 5 Halogenatomen,
oder
R⁶ für einen gegebenenfalls durch Halogen und/oder Alkyl mit 1 bis 4 Kohlenstoffatomen substituierten, heteroaromatischem Rest mit 5 oder 6 Ringgliedern und 1 oder 2 Heteroatomen steht,
oder
R⁶ für einen Rest der Formel 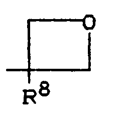 steht, worin
R⁸ für Alkyl mit 1 bis 4 Kohlenstoffatomen oder für Halogen steht,
durch Umsetzung von Benzylchloriden der Formel 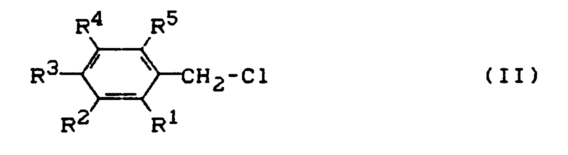 in welcher
R¹, R², R³, R⁴ und R⁵ die oben angegebenen Bedeutungen haben,
mit Säurechloriden der Formel
R⁶-CO-Cl (III)
in welcher
R⁶ die oben angegebene Bedeutung hat,
in Gegenwart von Zinkpulver und eines Palladium-Katalysators sowie in Gegenwart eines Verdünnungsmittels erhalt, dadurch gekennzeichnet, daß man
a) in einer ersten Stufe ein Benzylchlorid der Formel (II) mit einem Überschuß an Zinkpulver in Gegenwart eines Verdünnungsmittels bei Temperaturen zwischen 30°C und 200°C unter Schutzgasatmosphäre umsetzt, das überschüssige Zinkpulver abtrennt und dann
b) in einer zweiten Stufe das entstandene Benzylderivat der Formel 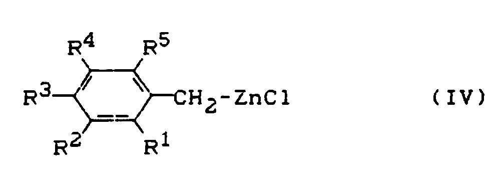
in welcher
R¹, R², R³, R⁴ und R⁵ die oben angegebenen Bedeutungen haben,
mit einem Säurechlorid der Formel (III) in Gegenwart eines Palladium-Katalysators und in Gegenwart eines Verdünnungsmittels bei Temperaturen zwischen 0°C und 150°C unter Schutzgasatmosphäre umsetzt.

2. Verfahren gemaß Anspruch 1, dadurch gekennzeichnet, daß man Benzylchloride der Formel (II) einsetzt, in denen
R¹, R², R³, R⁴ und R⁵ unabhängig voneinander für Wasserstoff, Fluor, Chlor, Brom, Alkyl mit 1 bis 4 Kohlenstoffatomen, Halogenalkyl mit 1 bis 4 Kohlenstoffatomen und 1 bis 5 Fluor- und/oder Chloratomen, Alkoxy mit 1 bis 4 Kohlenstoffatomen, Halogenalkoxy mit 1 bis 4 Kohlenstoffatomen und 1 bis 5 Fluor- und/oder Chloratomen, Cyano, gegebenenfalls einfach bis dreifach durch Fluor und/oder Chlor substituiertes Phenoxy, für Phenyl oder Naphthyl stehen, wobei jeder der beiden zuletzt genannten Reste einfach bis dreifach, gleichartig oder verschieden substituiert sein kann durch Fluor, Chlor Alkyl mit 1 bis 3 Kohlenstoffatomen, Halogenalkyl mit 1 oder 2 Kohlenstoffatomen und 1 bis 5 Fluor und/oder Chloratomen, Alkoxy mit 1 bis 3 Kohlenstoffatomen oder durch Halogenalkoxy mit 1 oder 2 Kohlenstoffatomen und 1 bis 5 Fluor- und/oder Chloratomen, oder
R¹, R², R³, R⁴ und R⁵ unabhängig voneinander für einen Rest der Formel
-COOR⁷
stehen, worin
R⁷ für Alkyl mit 1 bis 4 Kohlenstoffatomen, Phenyl oder Benzyl steht.

3. Verfahren gemäß Anspruch 1, dadurch gekennzeichnet, daß man Säurechloride der Formel (III) einsetzt, in denen
R⁶ für Alkyl mit 1 bis 6 Kohlenstoffatomen, Halogenalkyl mit 1 bis 6 Kohlenstoffatomen und 1 bis 6 Fluor-, Chlor- und/oder Bromatomen, Alkenyl mit 2 bis 8 Kohlenstoffatomen, Halogenalkenyl mit 2 bis 8 Kohlenstoffatomen und 1 bis 6 Fluor-, Chlor- und/oder Bromatomen, Alkoxycarbonylalkyl mit 1 bis 4 Kohlenstoffatomen im Alkylteil und 1 bis 4 Kohlenstoffatomen im Alkoxyteil, für Cycloalkyl mit 3 bis 7 Kohlenstoffatomen oder für Cycloalkenyl mit 5 bis 7 Kohlenstoffatomen steht, wobei die Cycloalkyl- und Cycloalkenyl-Reste einfach bis achtfach, gleichartig oder verschieden substituiert sein können durch Fluor, Chlor, Brom, Alkyl mit 1 bis 3 Kohlenstoffatomen, Halogenalkyl mit 1 bis 3 Kohlenstoffatomen und 1 bis 5 Fluor- und/oder Chloratomen, Alkoxy mit 1 bis 3 Kohlenstoffatomen, Halogenalkoxy mit 1 bis 3 Kohlenstoffatomen und 1 bis 5 Fluor- und/oder Chloratomen, durch Alkenyl mit 2 bis 4 Kohlenstoffatomen und/oder gegebenenfalls einfach oder zweifach, gleichartig oder verschieden durch Fluor, Chlor, Methyl und/oder Ethyl substituiertes Phenyl,
oder
R⁶ für Phenyl, Naphthyl, Phenylalkyl mit 1 bis 4 Kohlenstoffatomen im Alkylteil, Phenylalkenyl mit 2 oder 3 Kohlenstoffatomen im Alkenylteil, oder Phenoxyalkyl mit 1 bis 4 Kohlenstoffatomen im Alkylteil steht, wobei jeder dieser zuvor genannten Reste im Arylteil einfach bis dreifach, gleichartig oder verschieden substituiert sein kann durch Fluor, Chlor, Brom, Alkyl mit 1 bis 3 Kohlenstoffatomen, Halogenalkyl mit 1 bis 3 Kohlenstoffatomen und 1 bis 5 Fluor- und/oder Chloratomen, Alkoxy mit 1 bis 3 Kohlenstoffatomen und/oder Halogenalkoxy mit 1 bis 3 Kohlenstoffatomen und 1 bis 5 Fluor- und/oder Chloratomen,
oder
R⁶ für einen gegebenenfalls einfach oder zweifach, gleichartig oder verschieden durch Fluor, Chlor, Brom, Methyl und/oder Ethyl substituierten, heteroaromatischen Rest mit 5 oder 6 Ringgliedern und 1 oder 2 Stickstoff-, Sauerstoff- und/oder Schwefelatomen steht,
oder
R⁶ für einen Rest der Formel 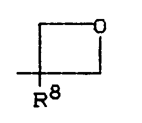 steht, worin
R⁸ für Methyl, Ethyl, Fluor, Chlor oder Brom steht.

4. Verfahren gemäß Anspruch 1, dadurch gekennzeichnet, daß man als Benzylchlorid der Formel (II) das 2-Chlor-benzylchlorid der Formel 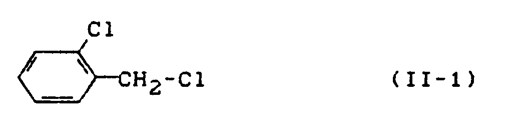 und als Säurechlorid der Formel (III) das 1-Chlorcyclopropan-carbonsäurechlorid der Formel 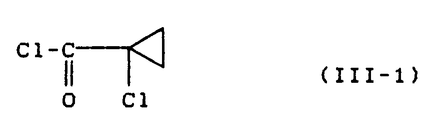 einsetzt.

5. Verfahren gemäß Anspruch 1, dadurch gekennzeichnet, daß man als Katalysator ein Palladium(II)-salz, einen Palladium(II)-komplex oder einen Palladium(O)-komplex einsetzt.

6. Verfahren gemäß Anspruch 1, dadurch gekennzeichnet, daß man auf 1 Mol Benzylchlorid der Formel (II) zwischen 1,05 und 3 Mol Zinkpulver einsetzt.

7. Verfahren gemäß Anspruch 1, dadurch gekennzeichnet, daß man auf 1 Mol Säurechlorid der Formel (III) zwischen 1,01 und 1,25 Mol Benzylchlorid der Formel (II) einsetzt.

8. Verfahren gemäß Anspruch 1, dadurch gekennzeichnet, daß man auf 1 Mol Säurechlorid der Formel (III) zwischen 0,0001 und 1 Mol-% an Palladiumkatalysator einsetzt.

9. Verfahren gemäß Anspruch 1, dadurch gekennzeichnet, daß man bei der Durchführung der ersten Stufe bei Temperaturen zwischen 50°C und 150°C arbeitet.

## Claims

1. Process for the preparation of benzyl ketones of the formula 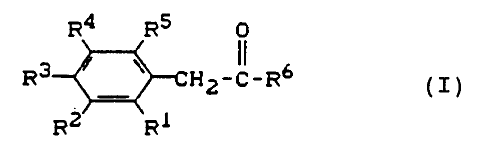 in which
R¹, R², R³, R⁴ and R⁵ independently of one another represent hydrogen, halogen, alkyl having 1 to 6 carbon atoms, halogenoalkyl having 1 to 6 carbon atoms and 1 to 13 halogen atoms, alkoxy having 1 to 6 carbon atoms, halogenoalkoxy having 1 to 6 carbon atoms and 1 to 13 halogen atoms, cyano or optionally halogen-substituted phenoxy, or represent aryl having 6 to 10 carbon atoms, it being possible for each of the aryl radicals to be substituted by halogen, alkyl having 1 to 4 carbon atoms, halogenoalkyl having 1 to 4 carbon atoms and 1 to 5 halogen atoms, alkoxy having 1 to 4 carbon atoms and/or halogenoalkoxy having 1 to 4 carbon atoms and 1 to 5 halogen atoms, or
R¹, R², R³, R⁴ and R⁵ independently of one another represent a radical of the formula
-COOR⁷
wherein
R⁷ represents alkyl having 1 to 6 carbon atoms, phenyl or benzyl,
and
R⁶ represents alkyl having 1 to 18 carbon atoms, halogenoalkyl having 1 to 18 carbon atoms and 1 to 12 halogen atoms, alkenyl having 2 to 18 carbon atoms or halogenoalkenyl having 2 to 18 carbon atoms and 1 to 12 halogen atoms; alkoxycarbonylalkyl having 1 to 6 carbon atoms in the alkyl part and 1 to 6 carbon atoms in the alkoxy part, or represents cycloalkyl having 3 to 8 carbon atoms, or represents cycloalkenyl having 5 to 8 carbon atoms, it being possible for each of the cycloalkyl and cycloalkenyl radicals to be substituted by halogen, alkyl having 1 to 4 carbon atoms, halogenoalkyl having 1 to 4 carbon atoms and 1 to 7 halogen atoms, alkoxy having 1 to 4 carbon atoms, halogenoalkoxy having 1 to 4 carbon atoms and 1 to 7 halogen atoms, alkenyl having 2 to 6 carbon atoms and/or phenyl which is optionally substituted by halogen and/or alkyl having 1 to 4 carbon atoms,
or
R⁶ represents aryl having 6 to 10 carbon atoms, or represents aralkyl having 6 to 10 carbon atoms in the aryl part and 1 to 4 carbon atoms in the alkyl part, or represents aralkenyl having 6 to 10 carbon atoms in the aryl part and 2 to 4 carbon atoms in the alkenyl part, or represents aroxyalkyl having 6 to 10 carbon atoms in the aryl part and 1 to 4 carbon atoms in the alkyl part, it being possible for each of these radicals to be substituted in the aryl part by halogen, alkyl having 1 to 6 carbon atoms, halogenoalkyl having 1 to 4 carbon atoms and 1 to 5 halogen atoms, alkoxy having 1 to 4 carbon atoms and/or halogenoalkoxy having 1 to 4 carbon atoms and 1 to 5 halogen atoms,
or
R⁶ represents a heteroaromatic radical which has 5 or 6 ring members and 1 or 2 hetero atoms and is optionally substituted by halogen and/or alkyl having 1 to 4 carbon atoms,
or
R⁶ represents a radical of the formula 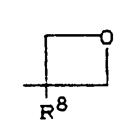 wherein
R⁸ represents alkyl having 1 to 4 carbon atoms, or represents halogen,
by reaction of benzyl chlorides of the formula 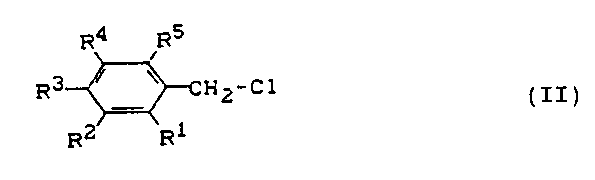 in which
R¹, R², R³, R⁴ and R⁵ have the abovementioned meanings,
with acid chlorides of the formula
R⁶-CO-Cl (III)
in which
R⁶ has the abovementioned meaning,
in the presence of zinc powder and a palladium catalyst and in the presence of a diluent, characterised in that
a) in a first stage a benzyl chloride of the formula (II) is reacted with an excess of zinc powder in the presence of a diluent at temperatures between 30°C and 200°C under an inert gas atmosphere, the excess zinc powder is separated off and then
b) in a second stage the benzyl derivative formed, of the formula 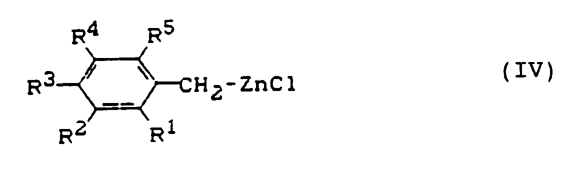
in which
R¹, R², R³, R⁴ and R⁵ have the abovementioned meanings,
is reacted with an acid chloride of the formula (III) in the presence of a palladium catalyst and in the presence of a diluent at temperatures between 0°C and 150°C under an inert gas atmosphere.

2. Process according to Claim 1, characterised in that benzyl chlorides of the formula (II) in which
R¹, R², R³, R⁴ and R⁵ independently of one another represent hydrogen, fluorine, chlorine, bromine, alkyl having 1 to 4 carbon atoms, halogenoalkyl having 1 to 4 carbon atoms and 1 to 5 fluorine and/or chlorine atoms, alkoxy having 1 to 4 carbon atoms, halogenoalkoxy having 1 to 4 carbon atoms and 1 to 5 fluorine and/or chlorine atoms, cyano or phenoxy which is optionally substituted by 1 to 3 fluorine and/or chlorine atoms, or represent phenyl or naphthyl, it being possible for each of the last two radicals mentioned to be substituted by 1 to 3 identical or different substituents from the group comprising fluorine, chlorine, alkyl having 1 to 3 carbon atoms, halogenoalkyl having 1 or 2 carbon atoms and 1 to 5 fluorine and/or chlorine atoms, alkoxy having 1 to 3 carbon atoms or halogenoalkoxy having 1 or 2 carbon atoms and 1 to 5 fluorine and/or chlorine atoms, or
R¹, R², R³, R⁴ and R⁵ independently of one another represent a radical of the formula
-COOR⁷
wherein
R⁷ represents alkyl having 1 to 4 carbon atoms, phenyl or benzyl,
are employed.

3. Process according to Claim 1, characterised in that acid chlorides of the formula (III) in which
R⁶ represents alkyl having 1 to 6 carbon atoms, halogenoalkyl having 1 to 6 carbon atoms and 1 to 6 fluorine, chlorine and/or bromine atoms, alkenyl having 2 to 8 carbon atoms or halogenoalkenyl having 2 to 8 carbon atoms and 1 to 6 fluorine, chlorine and/or bromine atoms, alkoxycarbonylalkyl having 1 to 4 carbon atoms in the alkyl part and 1 to 4 carbon atoms in the alkoxy part, or represents cycloalkyl having 3 to 7 carbon atoms, or represents cycloalkenyl having 5 to 7 carbon atoms, it being possible for the cycloalkyl and cycloalkenyl radicals to be substituted by one to eight identical or different substituents from the group comprising fluorine, chlorine, bromine, alkyl having 1 to 3 carbon atoms, halogenoalkyl having 1 to 3 carbon atoms and 1 to 5 fluorine and/or chlorine atoms, alkoxy having 1 to 3 carbon atoms, halogenoalkoxy having 1 to 3 carbon atoms and 1 to 5 fluorine and/or chlorine atoms, by alkenyl having 2 to 4 carbon atoms and/or phenyl which is optionally substituted by one or two identical or different substituents from the group comprising fluorine, chlorine, methyl and/or ethyl,
or
R⁶ represents phenyl, naphthyl, phenylalkyl having 1 to 4 carbon atoms in the alkyl part, phenylalkenyl having 2 or 3 carbon atoms in the alkenyl part, or phenoxyalkyl haying 1 to 4 carbon atoms in the alkyl part, it being possible for each of these previously mentioned radicals to be substituted in the aryl part by 1 to 3 identical or different substituents from the group comprising fluorine, chlorine, bromine, alkyl having 1 to 3 carbon atoms, halogenoalkyl having 1 to 3 carbon atoms and 1 to 5 fluorine and/or chlorine atoms, alkoxy having 1 to 3 carbon atoms and/or halogenoalkoxy having 1 to 3 carbon atoms and 1 to 5 fluorine and/or chlorine atoms,
or
R⁶ represents a heteroaromatic radical which has 5 or 6 ring members and 1 or 2 nitrogen, oxygen and/or sulphur atoms and is optionally substituted by one or two identical or different substituents from the group comprising fluorine, chlorine, bromine, methyl and/or ethyl,
or
R⁶ represents a radical of the formula 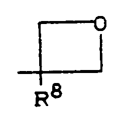 wherein
R⁸ represents methyl, ethyl, fluorine, chlorine or bromine,
are employed.

4. Process according to Claim 1, characterised in that the benzyl chloride of the formula (II) employed is 2-chloro-benzyl chloride of the formula 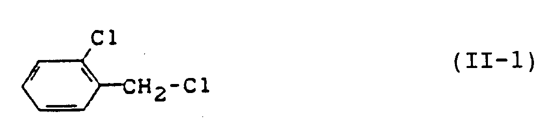 and the acid chloride of the formula (III) employed is 1-chlorocyclopropane-carbonyl chloride of the formula

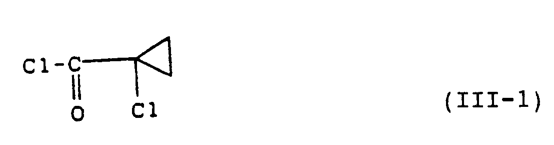

5. Process according to Claim 1, characterised in that a palladium(II) salt, a palladium(II) complex or a palladium(0) complex is employed as the catalyst.

6. Process according to Claim 1, characterised in that between 1.05 and 3 mol of zinc powder are employed per mole of benzyl chloride of the formula (II).

7. Process according to Claim 1, characterised in that between 1.01 and 1.25 mol of benzyl chloride of the formula (II) are employed per mole of acid chloride of the formula (III).

8. Process according to Claim 1, characterised in that between 0.0001 and 1 mol % of palladium catalyst is employed per mole of acid chloride of the formula (III).

9. Process according to Claim 1, characterised in that the first stage is carried out at temperatures between 50°C and 150°C.

## Revendications

1. Procédé pour la préparation de benzylcétones de formule 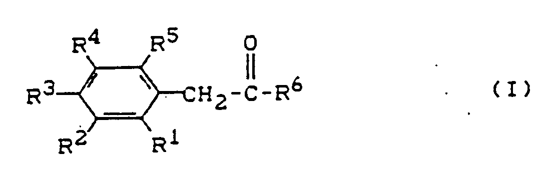 dans laquelle
R¹, R², R³, R⁴ et R⁵ représentent, indépendamment l'un de l'autre, un atome d'hydrogène, un atome d'halogène, un groupe alkyle contenant de 1 à 6 atomes de carbone, un groupe halogénalkyle contenant de 1 à 6 atomes de carbone et de 1 à 13 atomes d'halogène, un groupe alcoxy contenant de 1 à 6 atomes de carbone, un groupe halogénalcoxy contenant de 1 à 6 atomes de carbone et de 1 à 13 atomes d'halogène, un groupe cyano; un groupe phénoxy portant éventuellement un ou plusieurs substituants halogéno identiques ou différents; ou encore un groupe aryle contenant de 6 à 10 atomes de carbone, dans lequel chacun des radicaux aryle peut porter un ou plusieurs substituants identiques ou différents halogéno, alkyle contenant de 1 à 4 atomes de carbone, halogénalkyle contenant de 1 à 4 atomes de carbone et de 1 à 5 atomes d'halogène, alcoxy contenant de 1 à 4 atomes de carbone et/ou halogénalcoxy contenant de 1 à 4 atomes de carbone et de 1 à 5 atomes d'halogène, ou bien
R¹, R², R³, R⁴ et R⁵ représentent, indépendamment l'un de l'autre, un radical de formule
-COOR⁷
dans laquelle
R⁷ représente un groupe alkyle contenant de 1 à 6 atomes de carbone, un groupe phényle ou un groupe benzyle,
et
R⁶ représente un groupe alkyle contenant de 1 à 18 atomes de carbone, un groupe halogénalkyle contenant de 1 à 18 atomes de carbone et de 1 à 12 atomes d'halogène, un groupe alcényle contenant de 2 à 18 atomes de carbone, un groupe halogénalcényle contenant de 2 à 18 atomes de carbone et de 1 à 12 atomes d'halogène; un groupe alcoxycarbonylalkyle contenant de 1 à 6 atomes de carbone dans la fraction alkyle et de 1 à 6 atomes de carbone dans la fraction alcoxy; un groupe cycloalkyle contenant de 3 à 8 atomes de carbone ou encore un groupe cycloalcényle contenant de 5 à 8 atomes de carbone, dans lequel chacun des radicaux cycloalkyle et cycloalcényle peut porter un ou plusieurs substituants identiques ou différents halogéno, alkyle contenant de 1 à 4 atomes de carbone, halogénalkyle contenant de 1 à 4 atomes de carbone et de 1 à 7 atomes d'halogène, alcoxy contenant de 1 à 4 atomes de carbone, halogénalcoxy contenant de 1 à 4 atomes de carbone et de 1 à 7 atomes d'halogène, alcényle contenant de 2 à 6 atomes de carbone et/ou phényle portant éventuellement un ou plusieurs substituants identiques ou différents halogéno et/ou alkyle contenant de 1 à 4 atomes de carbone, ou bien
R⁶ représente un groupe aryle contenant de 6 à 10 atomes de carbone, un groupe aralkyle contenant de 6 à 10 atomes de carbone dans la fraction aryle et de 1 à 4 atomes de carbone dans la fraction alkyle; un groupe aralcényle contenant de 6 à 10 atomes de carbone dans la fraction aryle et de 2 à 4 atomes de carbone dans la fraction alcényle; ou encore un groupe aroxyalkyle contenant de 6 à 10 atomes de carbone dans la fraction aryle et de 1 à 4 atomes de carbone dans la fraction alkyle, dans lequel chacun de ces radicaux peut porter, dans la fraction aryle, un ou plusieurs substituants identiques ou différents halogéno, alkyle contenant de 1 à 6 atomes de carbone, halogénalkyle contenant de 1 à 4 atomes de carbone et de 1 à 5 atomes d'halogène, alcoxy contenant de 1 à 4 atomes de carbone et/ou halogénalcoxy contenant de 1 à 4 atomes de carbone et de 1 à 5 atomes d'halogène,
ou bien
R⁶ représente un radical hétéroaromatique contenant 5 ou 6 cycles et 1 ou 2 hétéroatomes, portant éventuellement un ou plusieurs substituants identiques ou différents halogéno et/ou alkyle contenant de 1 à 4 atomes de carbone,
ou bien
R⁶ représente un radical de formule 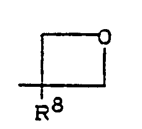 dans laquelle
R⁸ représente un groupe alkyle contenant de 1 à 4 atomes de carbone ou un atome d'halogène,
par mise en réaction de chlorures de benzyle répondant à la formule 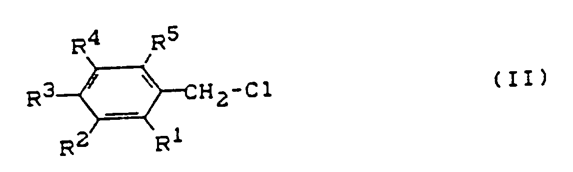 dans laquelle
R¹, R², R³, R⁴ et R⁵ ont les significations indiquées ci-dessus,
avec des chlorures d'acides de formule
R⁶-CO-Cl (III)
dans laquelle
R⁶ a la signification indiquée ci-dessus,
en présence de poudre de zinc et d'un catalyseur de palladium, de même qu'en présence d'un diluant, caractérisé en ce que
a) dans une première étape, on fait réagir un chlorure de benzyle de formule (II) avec un excès de poudre de zinc en présence d'un diluant, à des températures entre 30°C et 200°C, sous l'atmosphère d'un gaz de protection, on sépare la poudre de zinc en excès, et ensuite
b) dans une seconde étape, on fait réagir le dérivé benzylé obtenu répondant à la formule 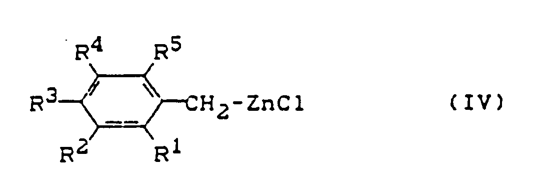 dans laquelle
R¹, R², R³, R⁴ et R⁵ ont les significations indiquées ci-dessus,
avec un chlorure d'acide de formule (III) en présence d'un catalyseur de palladium et en présence d'un diluant, à des températures entre 0°C et 150°C, sous l'atmosphère d'un gaz protecteur.

2. Procédé selon la revendication 1, caractérisé en ce qu'on met en oeuvre des chlorures de benzyle de formule (II), dans lesquels
R¹, R², R³, R⁴ et R⁵ représentent, indépendamment l'un de l'autre, un atome d'hydrogène, un atome de fluor, un atome de chlore, un atome de brome, un groupe alkyle contenant de 1 à 4 atomes de carbone, un groupe halogénalkyle contenant de 1 à 4 atomes de carbone et de 1 à 5 atomes de fluor et/ou de chlore, un groupe alcoxy contenant de 1 à 4 atomes de carbone, un groupe halogénalcoxy contenant de 1 à 4 atomes de carbone et de 1 à 5 atomes de fluor et/ou de chlore, un groupe cyano; un groupe phénoxy portant éventuellement de 1 à 3 substituants fluoro et/ou chloro identiques ou différents; un groupe phényle ou un groupe naphtyle, dans lequel chacun des deux radicaux mentionnés en dernier lieu peut porter de 1 à 3 substituants identiques ou différents fluoro, chloro, alkyle contenant de 1 à 3 atomes de carbone, halogénalkyle contenant 1 ou 2 atomes de carbone et de 1 à 5 atomes de fluor et/ou de chlore, alcoxy contenant de 1 à 3 atomes de carbone ou encore halogénalcoxy contenant 1 ou 2 atomes de carbone et de 1 à 5 atomes de fluor et/ou de chlore, ou bien
R¹, R², R³, R⁴ et R⁵ représentent, indépendamment l'un de l'autre, un radical de formule
-COOR⁷
dans laquelle
R⁷ représente un groupe alkyle contenant de 1 à 4 atomes de carbone, un groupe phényle ou un groupe benzyle.

3. Procédé selon la revendication 1, caractérisé en ce qu'on met en oeuvre des chlorures d'acides de formule (III), dans lesquels
R⁶ représente un groupe alkyle contenant de 1 à 6 atomes de carbone, un groupe halogénalkyle contenant de 1 à 6 atomes de carbone et de 1 à 6 atomes de fluor, de chlore et/ou de brome, un groupe alcényle contenant de 2 à 8 atomes de carbone, un groupe halogénalcényle contenant de 2 à 8 atomes de carbone et de 1 à 6 atomes de fluor, de chlore et/ou de brome; un groupe alcoxycarbonylalkyle contenant de 1 à 4 atomes de carbone dans la fraction alkyle et de 1 à 4 atomes de carbone dans la fraction alcoxy; un groupe cycloalkyle contenant de 3 à 7 atomes de carbone ou encore un groupe cycloalcényle contenant de 5 à 7 atomes de carbone, dans lequel les radicaux cycloalkyle et cycloalcényle peuvent porter de 1 à 8 substituants identiques ou différents fluoro, chloro, bromo, alkyle contenant de 1 à 3 atomes de carbone, halogénalkyle contenant de 1 à 3 atomes de carbone et de 1 à 5 atomes de fluor et/ou de chlore, alcoxy contenant de 1 à 3 atomes de carbone, halogénalcoxy contenant de 1 à 3 atomes de carbone et de 1 à 5 atomes de fluor et/ou de chlore, alcényle contenant de 2 à 4 atomes de carbone et/ou phényle portant éventuellement 1 ou 2 substituants identiques ou différents fluoro, chloro, méthyle et/ou éthyle,
ou bien
R⁶ représente un groupe phényle, un groupe naphtyle, un groupe phénylalkyle contenant de 1 à 4 atomes de carbone dans la fraction alkyle, un groupe phénylalcényle contenant 2 ou 3 atomes de carbone dans la fraction alcényle, ou encore un groupe phénoxyalkyle contenant de 1 à 4 atomes de carbone dans la fraction alkyle, dans lequel chacun de ces radicaux mentionnés ci-dessus peut porter, dans la fraction aryle, de 1 à 3 substituants identiques ou différents fluoro, chloro, bromo, alkyle contenant de 1 à 3 atomes de carbone, halogénalkyle contenant de 1 à 3 atomes de carbone et de 1 à 5 atomes de fluor et/ou de chlore, alcoxy contenant de 1 à 3 atomes de carbone et/ou halogénalcoxy contenant de 1 à 3 atomes de carbone et de 1 à 5 atomes de fluor et/ou de chlore,
ou bien
R⁶ représente un radical hétéroaromatique contenant 5 ou 6 cycles et 1 ou 2 atomes d'azote, d'oxygène et/ou de soufre, portant éventuellement 1 ou 2 substituants identiques ou différents fluoro, chloro, bromo, méthyle et/ou éthyle,
ou bien
R⁶ représente un radical de formule 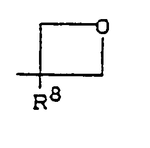 dans laquelle
R⁸ représente un groupe méthyle, un groupe éthyle, un atome de fluor, un atome de chlore ou un atome de brome.

4. Procédé selon la revendication 1, caractérisé en ce que, comme chlorure de benzyle de formule (II), on met en oeuvre le chlorure de 2-chlorobenzyle répondant à la formule 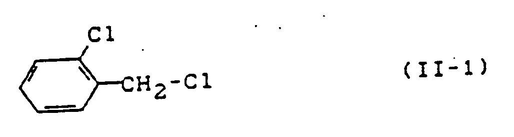 et, comme chlorure d'acide de formule (III), le chlorure d'acide 1-chlorocyclopropanecarboxylique de formule

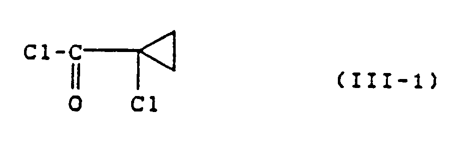

5. Procédé selon la revendication 1, caractérisé en ce que, comme catalyseur, on met en oeuvre un sel de palladium(II), un complexe de palladium(II) ou un complexe de palladium-(0).

6. Procédé selon la revendication 1, caractérisé en ce que, pour 1 mole de chlorure de benzyle de formule (II), on met en oeuvre entre 1,05 et 3 moles de poudre de zinc.

7. Procédé selon la revendication 1, caractérisé en ce que, pour 1 mole de chlorure d'acide de formule (III), on met en oeuvre entre 1,01 et 1,25 mole de chlorure de benzyle de formule (II).

8. Procédé selon la revendication 1, caractérisé en ce que, pour 1 mole de chlorure d'acide de formule (III), on met en oeuvre entre 0,0001 et 1 mole % de catalyseur de palladium.

9. Procédé selon la revendication 1, caractérisé en ce que, lors de la mise en oeuvre de la première étape, on travaille à des températures entre 50°C et 150°C.
